# FASCICULE DE BREVET EUROPEEN

(11) **EP 2 235 031 B1**
(45) Date de publication et mention de la délivrance du brevet: **26.07.2017**
(21) Numéro de dépôt: 09705572.7
(22) Date de dépôt: 22.01.2009
(51) Int. Cl.: C07H 15/224, A61K 31/7036, A61P 31/04

(54) **SYNTHESE DE NOUVEAUX DERIVES DE NEAMINE ET LEUR UTILISATION COMME AGENTS ANTIBACTERIENS**
SYNTHESE NEUARTIGER NEAMINDERIVATE UND IHRE VERWENDUNG ALS ANTIBAKTERIELLE WIRKSTOFFE
SYNTHESIS OF NOVEL NEAMINE DERIVATIVES AND USE THEREOF AS ANTIBACTERIAL AGENTS

(30) Priorité: 22.01.2008 FR 0850397
(43) Date de publication de la demande: 06.10.2010
(73) Titulaire: Université Joseph Fourier, 38041 Grenoble (FR)
(72) Inventeur: DECOUT, Jean-Luc, F-38410 Vaulnaveys le Haut (FR); BAUSSANE, Isabelle, F-38250 Lans en Vercors (FR); DESIRE, Jérôme, F-38570 Tencin (FR); PARIS, Jean-Marc, F-77360 Vaires sur Marne (FR)
(74) Mandataire: Cabinet Laurent & Charras
(86) Numéro de dépôt international: PCT/FR2009/050088
(87) Numéro de publication internationale: WO 2009/095588

(56) Documents cités:
- WO-A-2005/060573
- FR-A- 2 913 611
- US-A1- 2006 234 961
- T. JIKIHARA ET AL.: "Studies on aminosugars. XXXV. Synthesis of 3',4'-dideoxyneamine and 3'- and 4'-O-methylneamines" BULLETIN OF THE CHEMICAL SOCIETY OF JAPAN, vol. 46, 1973, pages 3507-3510, XP002500019
- T.L. NAGABHUSHAN, P.J.L.DANIELS: "Synthesis and biological properties of 6'-amino-6'-deoxygentamicin A" J. MED. CHEM., vol. 17, 1974, pages 1030-1031, XP002500020
- RIGUET E ET AL: "A route for preparing new neamine derivatives targeting HIV-1 TAR RNA" TETRAHEDRON, ELSEVIER SCIENCE PUBLISHERS, AMSTERDAM, NL, vol. 60, no. 37, 6 septembre 2004 (2004-09-06), pages 8053-8064, XP004527590 ISSN: 0040-4020
- M. LIU ET AL.: "Tethered bisubstrate derivatives as probes for mechanism and as inhibitors of aminoglycoside 3'-phosphotransferases" J. ORG. CHEM., vol. 65, 2000, pages 7422-7431, XP002500021
- D.H. RYU ET AL.: "Synthesis of (+),(-)-Neamine and their positional isomers as potential antibiotics" J. MED. CHEM., vol. 13, 2003, pages 901-903, XP002500022
- HADDAD J ET AL: "Design of novel antibiotics that bind to the ribosomal acyltransfer site" JOURNAL OF THE AMERICAN CHEMICAL SOCIETY, AMERICAN CHEMICAL SOCIETY, WASHINGTON, DC.; US, US, vol. 124, no. 13, 1 janvier 2002 (2002-01-01), pages 3229-3237, XP002208920 ISSN: 0002-7863
- JIAO G S ET AL: "Selectively guanidinylated derivatives of neamine. Syntheses and inhibition of anthrax lethal factor protease" BIOORGANIC & MEDICINAL CHEMISTRY LETTERS, PERGAMON, ELSEVIER SCIENCE, GB LNKD- DOI:10.1016/J.BMCL.2006.07.005, vol. 16, no. 19, 1 octobre 2006 (2006-10-01), pages 5183-5189, XP025107239 ISSN: 0960-894X [extrait le 2006-10-01]
- GREENBERG W A ET AL: "DESIGN AND SYNTHESIS OF NEW AMINOGLYCOSIDE ANTIBIOTICS CONTAINING NEAMINE AS AN OPTIMAL CORE STRUCTURE: CORRELATION OF ANTIBIOTIC ACTIVITY WITH IN VITRO INHIBITION OF TRANSLATION" JOURNAL OF THE AMERICAN CHEMICAL SOCIETY, AMERICAN CHEMICAL SOCIETY, NEW YORK, USA LNKD- DOI:10.1021/JA9910356, vol. 121, 1 janvier 1999 (1999-01-01), pages 6527-6541, XP002943570 ISSN: 0002-7863
- SUCHECK S J ET AL: "Design of small molecules that recognize RNA: development of aminoglycosides as potential antitumor agents that target oncogenic RNA sequences" ANGEWANDTE CHEMIE. INTERNATIONAL EDITION, WILEY VCH VERLAG, WEINHEIM LNKD- DOI:10.1002/(SICI)1521-3773(20000317)39:6< 1080::AID-ANIE1080>3.0.CO;2-B, vol. 39, no. 6, 1 janvier 2000 (2000-01-01), pages 1080-1084, XP002382469 ISSN: 1433-7851

## Description

### DOMAINE TECHNIQUE

La présente invention concerne le domaine des agents antibactériens.

Plus précisément, de nouveaux dérivés de la néamine ont été synthétisés et apparaissent comme très actifs.

### ETAT ANTERIEUR DE LA TECHNIQUE

Les aminoglycosides sont des pseudo-polysaccharides polyaminés naturels synthétisés par les bactéries Gram positif pour lutter contre d'autres bactéries. Les premiers composés de cette famille ont été découverts dans les années 40 et identifiés comme de puissants agents antibiotiques très vite utilisés à l'hôpital. La streptomycine, la néomycine, la kanamycine et la gentamicine furent les premières molécules de la famille des aminoglycosides à être isolées.

La majorité des aminoglycosides ont pour élément de structure commun le cycle streptamine ou 2-désoxystreptamine lié, par liaison glycosidique, à une ou deux unités mono ou disaccharidiques. Parmi eux sont illustrés ci-dessous la paromamine (**1**) et la néamine (**2**), constituées du cycle I (2-désoxystreptamine) lié sur la position 4 au cycle II par liaison glycosidique. Ces deux composés constituent les motifs de base de deux grandes familles d'aminoglycosides :
- la famille des dérivés substitués en position 5, à laquelle appartiennent la paromomycine (**3**) et la néomycine (**4**) ;
- la famille des dérivés (**5**) substitués en position 6, à laquelle appartiennent les kanamycines et la tobramycine.

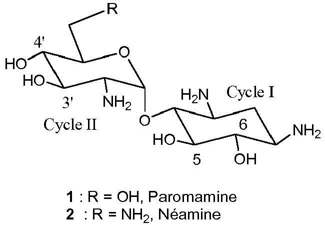

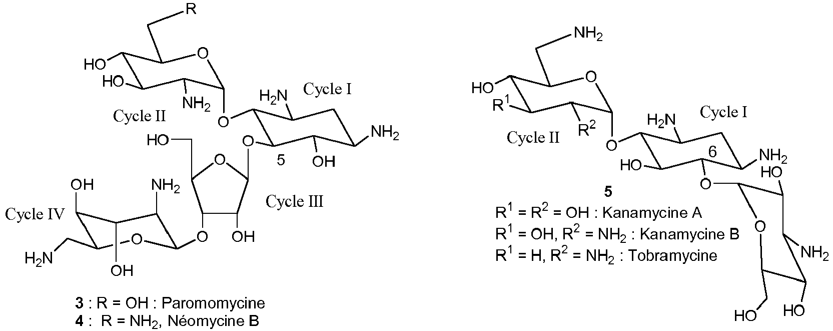

Parmi les agents antibiotiques actuels, les aminoglycosides représentent une classe très importante de molécules essentiellement utilisées en milieu hospitalier. L'utilisation restreinte dans ce milieu minimise le risque que des bactéries résistantes se développent, ce qui permet d'avoir accès à des molécules actives en cas d'urgence.

La plupart des aminoglycosides ont un effet antibiotique à la fois contre les bactéries Gram positif et Gram négatif. Ils sont souvent administrés en combinaison avec des β-lactames ou des fluoroquinolones pour élargir leurs spectres. En revanche, tous les aminoglycosides sont inactifs sur les bactéries anaérobies et les mycoplasmes.

Les aminoglycosides ont plusieurs modes d'action dont le principal réside dans la perturbation de la synthèse protéique en raison d'une interaction forte avec le site A de l'ARN ribosomal bactérien 16S. En 1987, Mozaed et Noller ont montré que certains aminoglycosides interagissent spécifiquement avec l'ARN ribosomal. Depuis cette découverte, d'autres ARNs pour lesquels les aminoglycosides sont de bons ligands ont été identifiés. Ils sont, par exemple, capables de se fixer aux ARN TAR, RRE et DIS du VIH-1.

Leurs effets antibiotiques sont également dus aux modifications des propriétés de la membrane bactérienne qu'ils provoquent.

Malheureusement, les aminoglycosides présentent certains inconvénients lors de leur utilisation comme médicaments. En effet, leur toxicité en limite l'utilisation thérapeutique, et comme pour de nombreux autres agents, des phénomènes de résistance apparaissent. Ainsi, les souches bactériennes résistantes sont responsables des maladies nosocomiales qui constituent un problème de santé majeur.

Ces résistances impliquent des modifications enzymatiques de la structure des aminoglycosides les rendant incapables de se lier à l'ARN ribosomal bactérien, la réduction de leur concentration intracellulaire par efflux, l'altération de la structure de la sous-unité 30S de l'ARN cible et la méthylation du site de fixation des aminoglycosides dans l'ARN.

En ce qui concerne les résistances causées par les enzymes de modification synthétisées par certaines bactéries, on rencontre trois classes d'enzymes de résistance :
- les aminosides *O*-phosphotransférases (APH) et les aminosides nucléotidyltransférases (ANT) qui phosphorylent les aminoglycosides sur les fonctions hydroxyles à l'aide de l'ATP ;
- les aminosides *N-*acétyltransférases (AAC) qui catalysent l'acylation des fonctions amines de l'aminoglycoside à l'aide du coenzyme A.

Les enzymes de modification sont spécifiques des différentes positions de l'aminoglycoside. Une enzyme qui phosphoryle la fonction hydroxyle en position 3' sur le cycle II va porter le nom APH3'. La kanamycine B est par exemple modifiée par les enzymes suivantes :

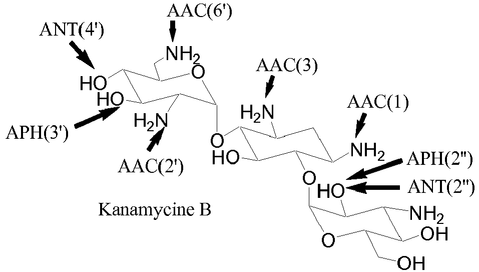

A noter qu'une enzyme bifonctionnelle AAC(6')-APH(2") a été découverte en 1997.

En ce qui concerne les mécanismes qui contribuent à la diminution de la concentration cellulaire en molécules actives, les pompes d'efflux présentes dans la membrane cellulaire des cellules procaryotes constituent un système de rejet des agents xénobiotiques qui risquent d'intoxiquer la cellule. Ces pompes d'efflux peuvent être suractivées à cause d'une mutation. Certaines cellules présentent des mutations dans les gènes de régulation de ces pompes de sorte qu'elles vont être surexprimées et que le rejet des xénobiotiques sera moins sélectif permettant l'efflux des aminoglycosides (systèmes « Multi-Drug Résistant » ou MDR).

Les effets antibiotiques majeurs des aminoglycosides et les problèmes de résistance ont donc conduit à un regain d'intérêt pour la chimie de ces molécules dans le but de trouver de nouveaux agents antibiotiques et/ou antiviraux. Il s'agit en particulier d'identifier de nouveaux agents actifs sur les bactéries résistantes aux aminoglycosides qui sont actuellement disponibles et donc aux traitements actuels. A ce titre, on peut citer par exemple le document US 2006/234961, qui décrit des dérivés de la paranmycine ayant un effet antibactérien, notamment sur des bactéries résistantes à certains aminoglycosides.

C'est dans cette démarche que s'inscrit la présente invention. En effet, le Demandeur a développé une nouvelle voie de synthèse des aminoglycosides, en particulier des dérivés de la néamine ou de la paromamine, et a mis en évidence que ces dérivés présentaient des propriétés antibiotiques remarquables.

### EXPOSE DE L'INVENTION

Ainsi et selon un premier aspect, l'invention concerne des compositions pharmaceutiques comprenant un composé présentant la formule suivante : 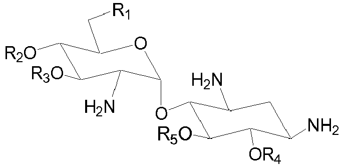 dans laquelle :
- R₁ = OH ou NH₂ ;
   R₂, R₃, R₄ et R₅, identiques ou différents, représentent : H ; un groupe alkyle, haloalkyle ou hétéroalkyle contenant entre 1 et 30 atomes de carbone en chaîne linéaire ou ramifiée ; ou un groupe alkaryle ou aralkyle contenant entre 1 et 30 atomes de carbone
   - si R₅ = H
      - si R₂ =R₃=R₄, alors R₂, R₃ et R₄ H ;
      - si R₂ = H, alors R₃ ≠ H et R₄ ≠ H ;
      - si R₃ = H, alors R₂ ≠ H et R₄ ≠ H ;
      - si R₄ = H, alors R₂ ≠ H et R₃ ≠ H ;
   - si R₅ ≠ H
      - si R₂ = H, alors R₃ ≠ H et R₄ ≠ H ;
      - si R₃ = H, alors R₂ ≠ H et R₄ ≠ H ;
      - si R₄ = H, alors R₂ ≠ H et R₃ ≠ H.

Selon un mode de réalisation privilégié, les compositions selon l'invention comprennent un composé présentant la formule suivante : 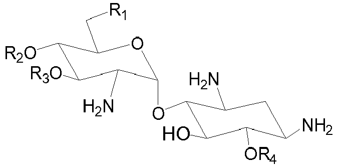 dans laquelle :
- R₁ = OH ou NH₂ ;
   R₂, R₃, R₄ et R₅, identiques ou différents, représentent : H ; un groupe alkyle, haloalkyle ou hétéroalkyle contenant entre 1 et 30 atomes de carbone en chaîne linéaire ou ramifiée ; ou un groupe alkaryle ou aralkyle contenant entre 1 et 30 atomes de carbone
- si R₂ =R₃=R₄, alors R₂, R₃ et R₄ ≠ H ;
- si R₂ = H, alors R₃ ≠ H et R₄ ≠ H ;
- si R₃ = H, alors R₂ ≠ H et R₄ ≠ H ;
- si R₄ = H, alors R₂ ≠ H et R₃ ≠ H.

Ainsi, la présente description divulgue des compositions pharmaceutiques comprenant des dérivés de la néamine ou de la paromamine disubstitués (3'+6 ou 3'+4' ou 4'+6) ou trisubstitués (3'+4'+6) aux positions 3', 4' et/ou 6. Sont également décrites des compositions pharmaceutiques comprenant des dérivés de la néamine ou de la paromamine trisubstitués impliquant la position 5 (3'+6+5 ou 3'+4'+5 ou 4'+6+5).

Dans la formule de la néamine et de la paromamine, OR₂, OR₃, OR₄ et OR₅ forment des fonctions alcool de formule OH.

Il est aussi décrit des composés tels que définis plus hauts dans lesquels, au moins deux, voire trois de ces fonctions alcool sont remplacées par des fonctions éther, ester, carbonate, carbamate, sulfonate ou sulfamate.

A noter que les composés décrits peuvent se présenter sous forme de sels, résultant de la réaction des fonctions amines avec un acide. Ainsi, les dérivés de la néamine (R₁ = NH₂) se présentent sous forme protonée et par exemple sous forme de tétrachlorhydrates ou de tétratrifluoroacétates.

Dans la fonction alcool, l'oxygène présente une liaison simple avec un atome d'hydrogène (OH).

Dans la fonction éther, l'oxygène présente une liaison simple avec un atome de carbone, lui-même engagé dans un groupement de type alkyle (OCRR'R") ou de type aryle (OAr).

Dans la fonction ester, l'oxygène présente une liaison simple avec un atome de carbone, lui-même engagé dans une double liaison avec un autre atome d'oxygène (OCOR).

Dans la fonction carbonate, l'oxygène présente une liaison simple avec un atome de carbone, lui-même engagé dans une double liaison avec un second atome d'oxygène et dans une autre liaison simple avec un troisième atome d'oxygène (OC(O)OR).

Dans la fonction carbamate, l'oxygène présente une liaison simple avec un atome de carbone, lui-même engagé dans une double liaison avec un autre atome d'oxygène et dans une liaison simple avec un atome d'azote OC(O)NHR ou OC(O)NRR').

Dans la fonction sulfonate, l'oxygène présente une liaison simple avec un atome de soufre, lui-même engagé dans deux doubles liaisons, chacune formée avec un atome d'oxygène, et dans une liaison simple avec un atome de carbone (OSO₂R).

Dans la fonction sulfamate, l'oxygène présente une liaison simple avec un atome de soufre, lui-même engagé dans deux doubles liaisons, chacune formée avec un atome d'oxygène, et dans une liaison simple avec un atome d'azote (OSO₂NHR ou OS0₂NRR').

La nature de ces fonctions découle du procédé mis en oeuvre pour la synthèse de ces dérivés, tel qu'il sera décrit ci-après.

Il apparaît clairement que dans les composés décrits, les fonctions OR₂, OR₃ et OR₄, et éventuellement OR₅ peuvent indépendamment être choisies dans le groupe constitué des fonctions alcool, éther, ester, carbonate, carbamate, sulfonate ou sulfamate. Toutes les combinaisons sont donc envisagées.

Sans vouloir être lié à une quelconque théorie, la modification et/ou l'encombrement de ces positions stratégiques par des substituants pourrait prévenir leur dégradation par les enzymes bactériennes de résistance impliquées dans les modifications de la structure des aminoglycosides. Ceci pourrait expliquer l'activité antibactérienne remarquable observée pour ces composés.

Ainsi, de préférence, dans les composés décrits, les résidus R₂, R₃, R₄ et R₅ identiques ou différents, représentent :
◆ H;
◆ un groupe alkyle, haloalkyle ou hétéroalkyle contenant entre 1 et 30 atomes de carbone en chaîne linéaire ou ramifiée ;
◆ un ou plusieurs groupes alcènyle ou alcynyle contenant entre 2 et 30 atomes de carbone en chaîne linéaire ou ramifiée ;
◆ un ou plusieurs groupes cycloalkyle, cycloalcènyle ou cycloalcynyle, substitués (tel que par exemple un noyau streptamine ou 2-deoxystreptamine) ou non, contenant entre 3 et 30 atomes de carbone en chaîne linéaire ou ramifiée;
◆ un ou plusieurs groupes aryle ou hétéroaryle contenant entre 3 et 10 atomes de carbone par cycle ;
◆ un groupe alkaryle ou aralkyle contenant entre 1 et 30 atomes de carbone, les termes aryle et alkyle ayant les définitions ci-dessus ;
◆ un ou plusieurs groupes alkoxy, thioalkyle, sulfonylalkyle, aminoalkyle contenant entre 1 et 30 atomes de carbone en chaîne linéaire ou ramifiée ;
◆ un ou plusieurs groupes alkoxyalkyle, alkylthioalkyle, alkylsulfonylalkyle, alkylaminoalkyle, alkylcétoalkyle, alkylester d'alkyle ou d'aryle contenant entre 1 et 30 atomes de carbone en chaîne linéaire ou ramifiée ;
◆ un ou plusieurs groupes hétérocyclique contenant entre 5 et 10 atomes de carbone par cycle ;
◆ un sucre, un oligosaccharide ou un pseudo-oligosaccharide tel que la néamine ou un de ses dérivés. A noter que les éventuels dérivés naturels entrant dans cette dernière catégorie et déjà décrits sont avantageusement exclus du champ de protection des composés en tant que tels ;
◆ un groupe alkylcarbonyle ou arylcarbonyle (-C(O)R), R étant défini comme R₂, R₃, R₄ et R₅ ;
◆ un groupe alkyloxycarbonyle ou aryloxycarbonyle (-C(O)OR), R étant défini comme R₂, R₃, R₄ et R₅ ;
◆ un groupe alkylcarbamoyle ou arylcarbamoyle (-C(O)NHR ou (-C(O)NRR'), R et R' identiques ou différents étant définis comme R₂, R₃, R₄ et R₅ ;
◆ un groupe sulfonyle (-SO₂R), R étant défini comme R₂, R₃, R₄ et R₅;
◆ un groupe aminosulfonyle (-SO₂NHR ou -SO₂NHRR'), R et R' identiques ou différents étant définis comme R₂, R₃, R₄ et R₅;
lesdits groupes étant tous éventuellement substitués par un ou plusieurs groupes nitro, cyano, hydroxy, carboxy, carbonyl ou amino, par un ou plusieurs halogènes ou par une ou plusieurs fonctions nitrile, cyanhydrine, aldéhyde.

Par "amine", on entend un groupement comportant un atome d'azote. Il peut s'agir d'une amine primaire NH₂, d'amines secondaires NHR' ou d'amines tertiaires NR'R". R' et R", identiques ou différents, sont définis comme R₂, R₃, R₄ et R₅.

Selon l'invention, le terme "alkyle" désigne un radical hydrocarboné linéaire ou ramifié de 1 à 30 atomes de carbone, tel que, à titre indicatif, méthyle, éthyle, propyle, isopropyle, butyle, tert-butyle, isobutyle, pentyle, hexyle, heptyle, octyle, nonyle, décyle, undécyle, dodécyle, tridécyle, tétradécyle, pentadécyle, hexadécyle, heptadécyle, octadécyle, nonadécyle ou icosyle. Le groupe alkyle ci-dessus défini peut comporter un ou plusieurs atomes d'halogène (fluor, chlore, brome ou iode). Dans ce cas, on parle de groupe "haloalkyle". Le groupe alkyle peut en outre comprendre des hétéroatomes choisis parmi P, O, N, S et Se. Dans ce cas, on parle de groupe "hétéroalkyle".

Par "alcényle", on entend une chaîne hydrocarbonée linéaire ou ramifiée de 2 à 30 atomes de carbone comprenant une ou plusieurs doubles liaisons. Des exemples de groupes alcényle sont les groupes alcényle portant une seule double liaison tels que -CH-CH=CH-CH₂, H₂C=CH- (vinyle) ou H₂C=CH-CH₂- (allyle).

Par "alcynyle", on entend une chaîne hydrocarbonée linéaire ou ramifiée de 2 à 30 atomes de carbone comprenant une ou plusieurs triples liaisons. Des exemples de groupes alcynyle sont les groupes alcynyle portant une seule triple liaison tel que -CH₂-C≡CH.

Le terme "cycloalkyle" désigne des groupements hydrocarbonés saturés qui peuvent être mono- ou polycycliques et comprennent de 3 à 10 atomes de carbone. Il s'agit, par exemple, de groupements cycloalkyle monocycliques tels que cyclopropyle, cyclobutyle, cyclopentyle, cyclohexyle, cycloheptyle, cyclooctyle, cyclononyle, cyclodécyle, cycloundécyle et cyclododécyle.

Par "cycloalcényle", on entend selon l'invention un groupe dérivé d'un groupe cycloalkyle tel que défini ci-dessus, présentant une ou plusieurs doubles liaisons, par exemple deux doubles liaisons. Il s'agit par exemple du groupement cyclohexène (une double liaison) ou cyclopenta-1,3-diène (deux doubles liaisons).

Par "cycloalcynyle", on entend selon l'invention un groupe dérivé d'un groupe cycloalkyle tel que défini ci-dessus, présentant une ou plusieurs triples liaisons, par exemple une triple liaison.

Le terme "aryle" représente un groupement hydrocarboné monocyclique ou polycyclique aromatique comprenant 3 à 10 atomes de carbone par cycle, tel que phényle ou naphtyle. Le terme "hétéroaryle" désigne un groupe aromatique monocyclique ou polycyclique comprenant entre 3 et 10 atomes de carbone par cycle et comprenant 1, 2 ou 3 hétéroatomes endocycliques par cycle choisis parmi P, O, N, S et Se. Des exemples en sont les groupes furyle, thiényle, pyrrolyle, oxazolyle, isoxazolyle, thiazolyle, isothiazolyle, imidazolyle, pyrazolyle, oxadiazolyle, triazolyle, thiadiazolyle, pyridyle, pyridazinyle, pyrazinyle et triazinyle.

Par "alkaryle", on entend un groupe alkyle, substitué par un groupe aryle, ces deux groupes étant définis ci-dessus.

Par "aralkyle", on entend un groupe aryle, substitué par un groupe alkyle, ces deux groupes étant définis ci-dessus.

Par "alkoxy", on entend un groupe *O*-alkyle ayant de 1 à 30 atomes de carbone, notamment méthoxy, éthoxy, propoxy et butoxy. Par "alkoxyalkyle", on entend un groupe alkyle-*O*-alkyle ayant de 1 à 30 atomes de carbone. Les groupes "thioalkyle" ou "alkylthioalkyle", "sulfonylalkyle" ou "alkylsulfonylalkyle" et "aminoalkyle" ou "alkylaminoalkyle" comportent en outre respectivement un ou plusieurs atomes de soufre, un ou plusieurs groupes sulfonyl et une ou plusieurs fonctions amine.

Le terme "groupe hétérocyclique" désigne des cycles carbonés saturés ou insaturés, monocycliques ou polycycliques, présentant 1, 2 ou 3 hétéroatomes endocycliques choisis parmi P, O, N, S et Se. Ce sont généralement des dérivés des groupes hétéroaryles décrits ci-dessus. Des exemples d'hétérocycles insaturés sont dihydrofuryle, dihydrothiényle, dihydropyrrolyle, pyrrolinyle, oxazolinyle, thiazolinyle, imidazolinyle, pyrazolinyle, isoxazolinyle, isothiazolinyle, oxadiazolinyle, pyranyle et les dérivés mono- insaturés de la pipéridine, du dioxane, de la pipérazine, du trithiane, de la morpholine du dithiane, de la thiomorpholine, ainsi que tétrahydropyridazinyle, tétrahydropyrimidinyle, et tétrahydrotriazinyle.

En raison des procédés avantageusement mis en oeuvre dans le cadre de la présente invention, dans les composés décrits, R₄ (en position 6) et R₃ (en position 3'), éventuellement R₂ (en position 4'), et éventuellement R₅ (en position 5), sont de préférence identiques.

De préférence, la composition de l'invention comprend un composé tel que défini plus haut dans lequel R₂ et R₃, éventuellement R₄ et éventuellement R₅, sont identiques.

De préférence, dans les composés décrits, R₂ et/ou R₃ et/ou R₄ et/ou R₅ sont choisis dans le groupe suivant : naphtyl-2-méthylène (2NM), naphtyl-1-méthylène (1NM), et hexyle.

De préférence, la composition de l'invention comprend un composé tel que défini plus haut dans lequel R₂ et/ou R₃ et/ou R₄ et/ou R₅ sont choisis dans le groupe suivant : naphtyl-2-méthylène, naphtyl-1-méthylène, et hexyle.

Des composés disubstitués ou trisubstitués particulièrement privilégiés compris dans la composition pharmaceutique selon l'invention sont les suivants :

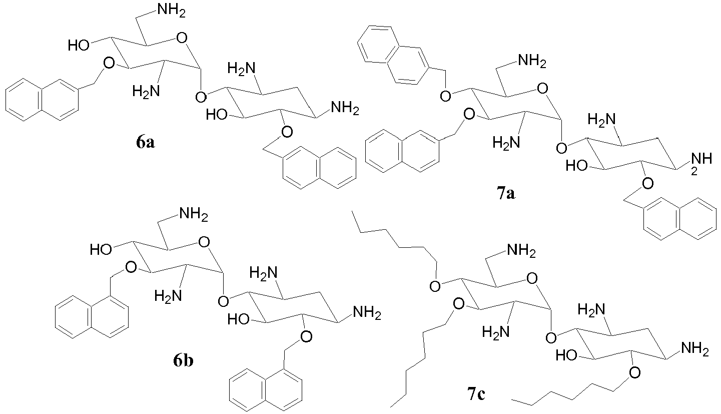

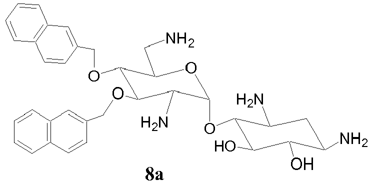

Le composé **6a** correspond à la 3',6-*O,O*'-di(naphtyl-2-méthylène) néamine.
Le composé **7a** correspond à la 3',4',6-*O,O',O*"-tri(naphtyl-2-méthylène) néamine.
Le composé **6b** correspond à la 3', 6-*O,O*'-di(naphtyl-1-méthylène) néamine.
Le composé **7c** correspond à la 3',4',6-*O,O',O*"-tri-*n*-hexyl néamine.
Le composé **8a** correspond à la 3,4'-*O,O*'-di-(naphtyl-2-méthylène) néamine.

Plus généralement et en relation avec le dérivé 2NM, des composés présentant les formules suivantes ont été aisément préparés et présentent l'activité biologique recherchée explicitée ci-dessous :

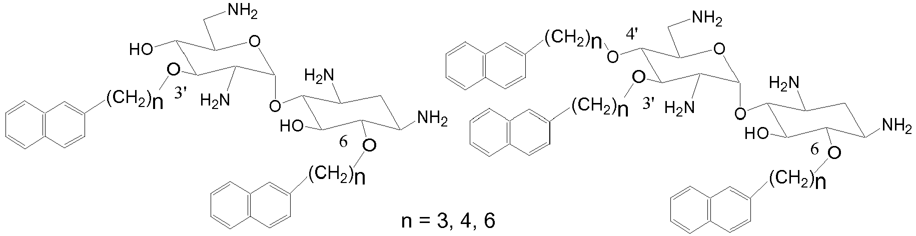

On peut citer notamment les composés suivants :
- 3',6-*O,O*'-di(naphtyl-2-propyl) néamine ;
- 3',4',6-*O,O',O*"-tri(naphtyl-2-propyl) néamine ;
- 3',6-*O,O*'-di(naphtyl-2-butyl) néamine ;
- 3',4',6-*O,O',O*"-tri(naphtyl-2-butyl) néamine.

Ainsi, de préférence, la composition selon l'invention comprend un composé ayant une formule choisie dans le groupe suivant : 3',6-O,O'-di(naphtyl-2-méthylène) néamine; 3',4',6-O,O',O"-tri(naphtyl-2-méthylène) néamine; 3',6-O,O'-di(naphtyl-1-méthylène) néamine; 3',4',6-O,O',O"-tri-n-hexyl néamine; 3',4'-O,O'-di-(naphtyl-2-méthylène) néamine; 3',6-O,O'-di(naphtyl-2-propyl) néamine; 3',4',6-O,O',O"-tri(naphtyl-2-propyl) néamine; 3',6-O,O'-di(naphtyl-2-butyl) néamine et 3',4',6-O,O',O"-tri(naphtyl-2-butyl) néamine.

Il a été montré, dans le cadre de la présente invention, que les composés tels que décrits ci-dessus pouvaient avoir un intérêt dans le cadre d'une application thérapeutique. C'est pourquoi et selon un autre aspect, la présente invention vise une composition selon l'invention pour son utilisation comme médicament.

Dans la composition de l'invention, le composé se présente avantageusement sous forme de sel pharmaceutiquement acceptable, notamment chlorhydrate ou méthylsulfonate.

Bien sûr, une telle composition peu également contenir tout excipient ou véhicule pharmaceutiquement inerte, et éventuellement un ou plusieurs autres principes actifs. Dans le cadre des cocktails d'antibiotiques, un principe actif privilégié est un antibiotique d'une autre classe, préférentiellement un β-lactame ou une fluoroquinolone. Ainsi et selon un autre aspect, la composition selon l'invention comprend au moins un autre antibiotique tel qu'un β-lactame ou une fluoroquinolone.

Comme déjà dit, la composition selon l'invention sert avantageusement à la préparation de médicaments destinés à la prophylaxie ou au traitement des infections bactériennes. Ces infections bactériennes peuvent être dues aussi bien à des bactéries Gram positif, telles que *Staphylococcus aureus,* que des bactéries Gram négatif, telles qu'*Escherichia coli, Pseudomonas aeriginosa, Acinetobacter lwoffi, Klebsiella pneumoniae*, *Enterobacter aerogenes, Citrobacter amalonaticus,* sauvages or résistantes. De manière remarquable, les compositions selon l'invention peuvent être actives aussi bien vis-à-vis de souches sensibles, que vis à vis de souches considérées comme résistantes notamment aux aminoglycosides classiques tels que la néamine ou la néomycine. Ainsi et selon un autre aspect, l'invention vise une composition telle que définie pour son utilisation dans le traitement des infections bactériennes liées aux bactéries Gram positif telles que *Staphylococcus aureus* ou Gram négatif telles qu'*Escherichia coli*, notamment les souches résistantes aux aminoglycosides.

Il est aussi décrit des procédés de synthèse permettant d'obtenir, à partir de la néamine ou de la paromamine, les dérivés di- ou tri-substitués aux positions d'intérêt.

Selon un autre aspect, la présente invention concerne également le procédé de synthèse permettant d'obtenir, à partir de la néamine ou de la paromamine, des composés de formule : 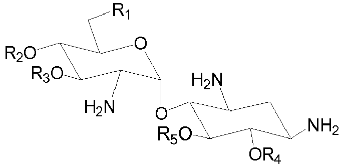 dans laquelle :
- R₁ = OH ou NH2 ;
- R₂, R₃, R₄ et R₅, identiques ou différents, représentent : H ; un groupe alkyle, haloalkyle ou hétéroalkyle contenant entre 1 et 30 atomes de carbone en chaîne linéaire ou ramifiée ; ou un groupe alkaryle ou aralkyle contenant entre 1 et 30 atomes de carbone;
   - si R₅ = H
      - si R₂ =R₃=R₄, alors R₂, R₃ et R₄ ≠ H ;
      - si R₂ = H, alors R₃ ≠ H et R₄ ≠ H ;
      - si R₃ = H, alors R₂ ≠ H et R₄ ≠ H ;
      - si R₄ = H, alors R₂ ≠ H et R₃ ≠ H ;
   - si R₅ ≠ H
      - si R₂ = H, alors R₃ ≠ H et R₄ ≠ H ;
      - si R₃ = H, alors R₂ ≠ H et R₄ ≠ H ;
      - si R₄ = H, alors R₂ ≠ H et R₃ ≠ H.

Ainsi, différentes voies de synthèse ont été mises en évidence dans le cadre de l'invention. Ces différentes voies ont en commun :
- la substance de départ qui est la paromamine R₁ = OH en position 6') ou la néamine (R₁ = NH₂ en position 6') ;
- une première étape consistant en la protection des fonctions alcool et/ou amines en positions 1, 3, 2', 6', avantageusement par tritylation ;
- puis une modification du groupement hydroxyle en position 6 et/ou 3' ;
- et une étape finale de déprotection des positions 1, 3, 2' et 6'.

En d'autres termes, le procédé visé comprend les étapes suivantes :
- protection des fonctions alcool et/ou amines en position 1, 3, 2' et 6', avantageusement par tritylation, sur les molécules paromamine ou néamine ;
- modification de la fonction hydroxyle en position 6 et/ou 3' ;
- déprotection, avantageusement par détritylation, des fonctions en positions 1, 3, 2' et 6'.

La première étape est bien connue puisqu'il s'agit de protéger les fonctions, notamment amine, aux positions 1, 3, 2' et 6'. Selon un procédé décrit dans le document WO 2005/060573, cette étape peut être réalisée à l'aide de groupements trityle (triphénylméthyle), 4-monométhoxytrityle, 4',4'-diméthoxytrityle ou 4,4',4"-triméthoxytrityle), en présence d'une base qui a un pKₐ supérieur à celui des fonctions amines présentes à ces positions, si celle-ci sont protonées dans le dérivé de départ, la paromamine ou la néamine.

L'étape finale d'un tel procédé permettant d'aboutir aux dérivés recherchés est également connue en soi puisqu'il s'agit d'une déprotection des fonctions amines et hydroxyles par traitement en milieu acide, par exemple avec un mélange TFA/anisole.

Les étapes intermédiaires ont pour but d'introduire les substituants aux positions visées, notamment par alkylation. En fonction de la réactivité des groupements aux différentes positions, des étapes transitoires de protection peuvent être nécessaires.

Selon une première voie de synthèse (voie A), le produit protégé réagit avec un dérivé RX halogéné (par exemple X = Cl, Br ou I) ou sulfonylé (par exemple X = mésyl ou tosyl), portant le groupement R à introduire en position 6, éventuellement en position 3', et éventuellement en position 4'. R répond à la même définition que celle donnée pour R₂, R₃ et R₄ dans le cadre de la présente invention.

Cette réaction se déroule en présence d'une base capable de déprotoner les fonctions hydroxyles visées, par exemple l'hydrure de sodium. La température, le solvant, la nature du dérivé halogéné ou sulfonylé et les proportions de réactifs sont choisis en fonction de la sélectivité recherchée (alkylation en position 6 et/ou 3' et/ou 4'). Le *N,N-*diméthylformamide (DMF) est le solvant généralement utilisé mais l'utilisation par exemple d'un mélange DMF- tétrahydrofurane (THF) ralentit la réaction et peut améliorer la sélectivité recherchée. Dans certains cas, de l'iodure de tétrabutylammonium peut être ajouté au milieu réactionnel pour accélérer la réaction.

Dans ce cas de figure, on obtient simultanément des dérivés monosubstitués en 6, disubstitués en 3'+6 et trisubstitués en 3'+4'+6. En outre et selon le schéma réactionnel le plus simple, ces dérivés présentent des résidus R₃ (position 3'), R₄ (position 6) et R₂ (position 4'), identiques.

Le schéma réactionnel correspondant est représenté ci-dessous en rapport avec la néamine tétratritylée **9** :

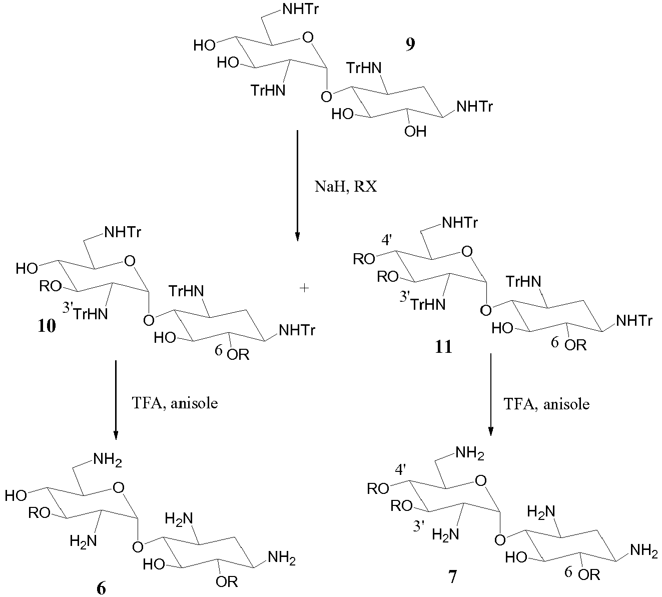

Il a été observé que de manière tout à fait inattendue, l'étape finale de détritylation en milieu acide du dérivé **11a** utilisée pour préparer le dérivé **7a** permettait également d'obtenir le dérivé **8a**, alkylé en positions 3'+4', lorsque le temps de réaction est augmenté (24 heures). Le schéma réactionnel correspondant est présenté ci-dessous :

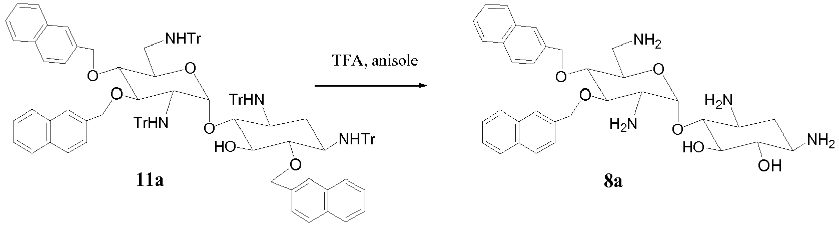

Ces dérivés se sont avérés actifs comme agents antibactériens.

Le dérivé monosubstitué en 6 est, quand à lui, très intéressant car il sert de produit intermédiaire dans deux des autres voies de synthèse (voies B et C).

Selon un second mode de réalisation (voie B), le dérivé monosubstitué en 6 portant le groupement R est mis en réaction avec un dérivé R'X halogéné (par exemple X = Cl, Br, I) ou sulfonylé (par exemple X = mésyl ou tosyl), portant le groupement R' à introduire en position 3', et éventuellement en position 4'. Il est envisageable d'utiliser un composé R"X distinct pour la position 4'. A nouveau, R' et R" répondent à la même définition que celle donnée pour R₂, R₃ et R₄ dans le cadre de la présente invention.

A l'issue de cette réaction, on obtient simultanément des dérivés disubstitués en 3'+6 et trisubstitués en 3'+4'+6, présentant des résidus R₃ (position 3') et R₄ (position 6) éventuellement différents, et des résidus R₃ (position 3') et R₂ (position 4') identiques ou éventuellement différents.

Ces réactions se déroulent en présence d'une base capable de déprotoner les fonctions hydroxyles visées, par exemple l'hydrure de sodium. La température, le solvant, la nature du dérivé halogéné ou sulfonylé et les proportions de réactifs sont choisis en fonction de la sélectivité recherchée (alkylation en position 6 et/ou 3' et/ou 4'). Le *N*,*N-*diméthylformamide (DMF) est le solvant généralement utilisé mais l'utilisation par exemple d'un mélange DMF-tétrahydrofurane (THF) ralentit la réaction et peut améliorer la sélectivité recherchée. Dans certains cas, de l'iodure de tétrabutylammonium peut être ajouté au milieu réactionnel pour accélérer la réaction.

Le schéma réactionnel correspondant est représenté ci-dessous en rapport avec la néamine tétratritylée :

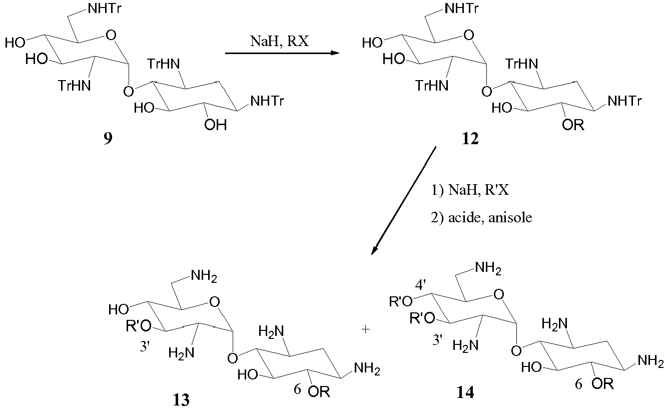

Selon un mode de réalisation alternatif (voie C), le dérivé monosubstitué en 6 portant le groupement R est d'abord protégé en position 3', à l'aide d'un groupement protecteur (Z), puis mis en réaction avec un dérivé R'X halogéné (par exemple X = Cl, Br ou I) ou sulfonylé (par exemple X = mésyl ou tosyl), portant un groupement R' à introduire en position 4'. R' répond à la même définition que celle donnée pour R₂, R₃ et R₄ dans le cadre de la présente invention.

La protection en position 3' peut se faire avec un groupement Z acido-labile par réaction avec un dérivé halogéné (par exemple X = Cl, Br ou I) ou sulfonylé (par exemple X = mésyl ou tosyl), notamment de type arylméthylène (ArCH₂X, Ar = 2-naphtyl, 1-naphtyl, anthracényl, fluorényl, pyrényl ...) ou silylé (Z = *tert*-butyldiméthylsilyl, triéthylsilyl...).

Les réactions se déroulent en présence d'une base capable de déprotoner les fonctions hydroxyles visées, par exemple l'hydrure de sodium. La température, le solvant, la nature du dérivé halogéné ou sulfonylé, et les proportions de réactifs sont choisis en fonction de la sélectivité recherchée (protection par alkylation en position 3' pour introduire un groupement protecteur acido-labile puis alkylation en position 4'). Le *N*,*N-*diméthylformamide (DMF) est le solvant généralement utilisé mais l'utilisation par exemple d'un mélange DMF-tétrahydrofurane (THF) ralentit la réaction et peut améliorer la sélectivité recherchée. Dans certains cas, de l'iodure de tétrabutylammonium peut être ajouté au milieu réactionnel pour accélérer la réaction.

A l'issue de cette réaction, on obtient des dérivés disubstitués en 4'+6, présentant des résidus R₂ (position 4') et des résidus R₄ (position 6) éventuellement différents.

Le schéma réactionnel correspondant est représenté ci-dessous en rapport avec la néamine tétratritylée :

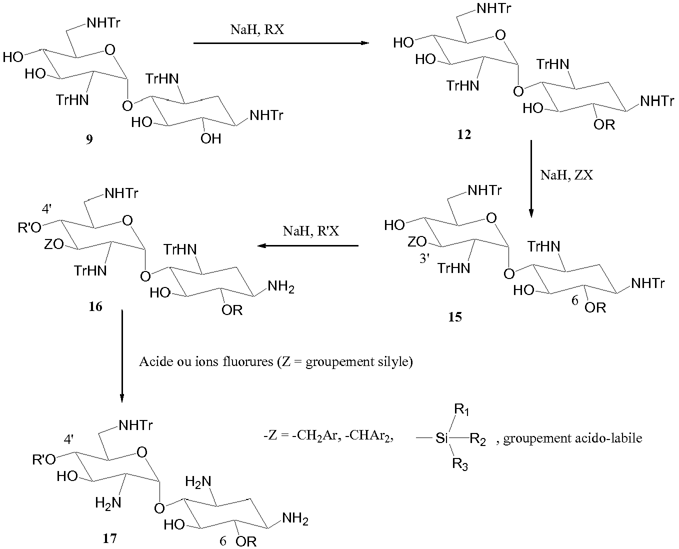

Enfin, selon un dernier mode de réalisation, la modification en position 6 consiste à introduire un groupe protecteur (Z). Cette protection peut se faire avec un groupement Z acido-labile, par réaction avec un dérivé halogéné (par exemple X = Cl, Br ou I) ou sulfonylé (par exemple X = mésyl ou tosyl), notamment de type arylméthylène (ArCH₂X, Ar = 2-naphtyl, 1-naphtyl, anthracényl, fluorényl, pyrényl...) ou silylé (Z = *test-*butyldiméthylsilyl, triéthylsilyl...).

Le dérivé protégé est alors mis en réaction avec un dérivé RX halogéné (par exemple X = Cl, Br ou I) ou sulfonylé (par exemple X = mésyl ou tosyl) portant le groupement R à introduire en position 3' et en position 4'. R répond à la même définition que celle donnée pour R₂, R₃ et R₄ dans le cadre de la présente invention.

Ces réactions se déroulent en présence d'une base capable de déprotoner les fonctions hydroxyles visées, par exemple l'hydrure de sodium. La température, le solvant, la nature du dérivé halogéné ou sulfonylé, et les proportions de réactifs sont choisis en fonction de la sélectivité recherchée (alkylations identiques ou différentes en position 3' et 4' avec une première alkylation en 3'). Le *N,N-*diméthylformamide (DMF) est le solvant généralement utilisé mais l'utilisation par exemple d'un mélange DMF-tétrahydrofurane (THF) ralentit la réaction et peut améliorer la sélectivité recherchée. Dans certains cas, de l'iodure de tétrabutylammonium peut être ajouté au milieu réactionnel pour accélérer la réaction.

A l'issue de cette deuxième étape et selon le schéma réactionnel le plus simple, on obtient des dérivés disubstitués en 3'+4', présentant des résidus R₃ (position 3') et R₂ (position 4') identiques. Toutefois, ces résidus peuvent être différents si l'alkylation se fait en deux temps : une première alkylation en position 3' avec un résidu R donné, puis une seconde étape d'alkylation en position 4' avec un résidu R' différent de R.

Le schéma réactionnel correspondant est représenté ci-dessous en rapport avec la néamine tétratritylée :

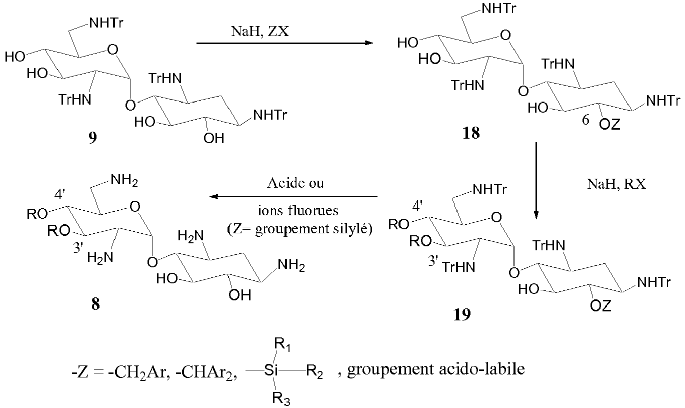

Une autre voie de synthèse des composés tels que définis ci-dessus consiste à mettre en oeuvre un procédé biphasique en présence d'un agent de transfert de phase. Par rapport aux procédés en solution décrit ci-dessous, ce type de procédé présente une sélectivité très importante, donnant lieu à un produit très majoritaire dont la nature dépend des conditions expérimentales mises en oeuvre.

Ainsi et en fonction de la nature de l'agent de transfert de phase en présence, deux grandes voies de synthèse ont été suivies, l'une basée sur la modification de la fonction hydroxyle en 3' et l'autre sur la modification de la fonction hydroxyle en 6 :

Selon un premier aspect, l'alkylation se fait sélectivement en 3' (voie de droite sur le schéma ci-dessous), voire en 3'+6 (voie de gauche sur le schéma ci-dessous), en fonction de la quantité d'halogénure d'alkyle (RX) en présence.

L'alkylation se fait par transfert de phase avec une phase aqueuse contenant de la soude (30 à 50%) et une phase organique composée de toluène (ou par exemple le dichlorométhane) et contenant le dérivé **9** dissous. L'agent de transfert de phase, en l'occurrence l'iodure ou le bromure de tétrabutylammonium, est ensuite ajouté puis l'halogénure d'alkyle (RX), par exemple le chlorure de para-méthoxybenzyle (PMBCl). Dans une première condition qui consiste à limiter la quantité d'halogénure d'alkyle (par exemple PMBCl) et la quantité d'agent de transfert de phase, il est possible de privilégier la formation du dérivé fonctionnalisé en position 3'. La réaction peut être réalisée à température ambiante ou à température plus élevée. Après isolement du dérivé 3' formé, celui peut être bisalkylé dans des conditions différentes en position 4' et 6 puis déprotégé en présence de TFA et d'anisole pour conduire au dérivé bisalkylé en position 4' et 6. Alternativement, il peut être trisalkylé dans d'autres conditions en position 4', 5 et 6 puis déprotégé en présence de TFA et d'anisole pour conduire au dérivé trisalkylé en position 4', 5 et 6.

Dans une autre condition qui consiste à ne réaliser que la première étape d'alkylation par transfert de phase et à utiliser une quantité plus importante d'halogénure d'alkyle (par exemple PMBCl, 1NMBr, 2NMBr), il est possible de privilégier la formation du dérivé bisalkylé en position 3',6.

Si le groupement introduit préalablement en position 3' est stable dans le mélange TFA/anisole, des dérivés 3',4',6 trialkylés mixtes (R≠ R') peuvent également être préparés après déprotection.

Ces différentes voies sont représentées sur le schéma ci-dessous :

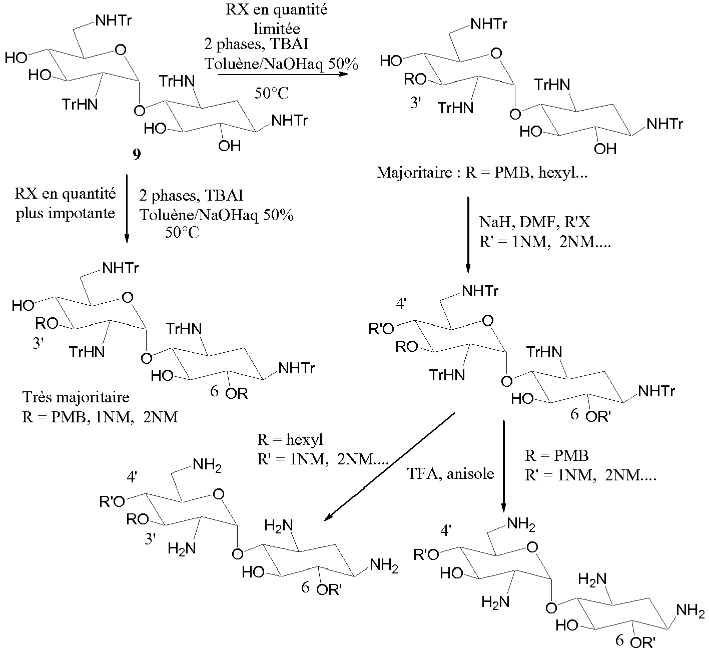

Ce schéma peut par exemple servir à la synthèse du :
- dérivé 4',6-di-2NM de la néamine= 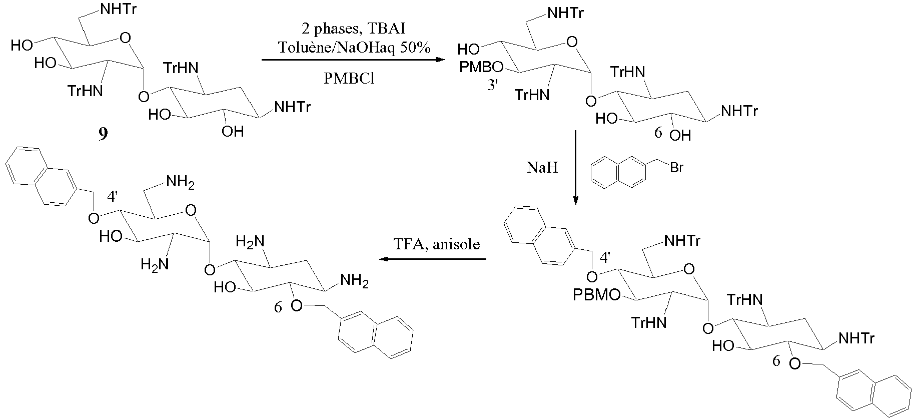
- dérivé 3',6-di-2NM en série paromamine= 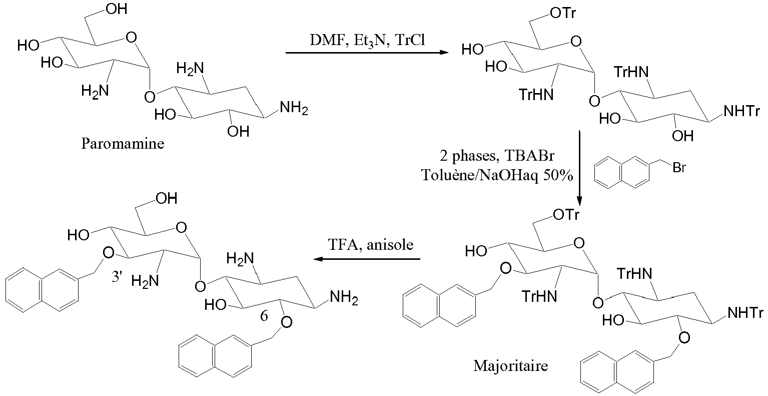
- dérivé 3',6-di-1NM en série néamine=

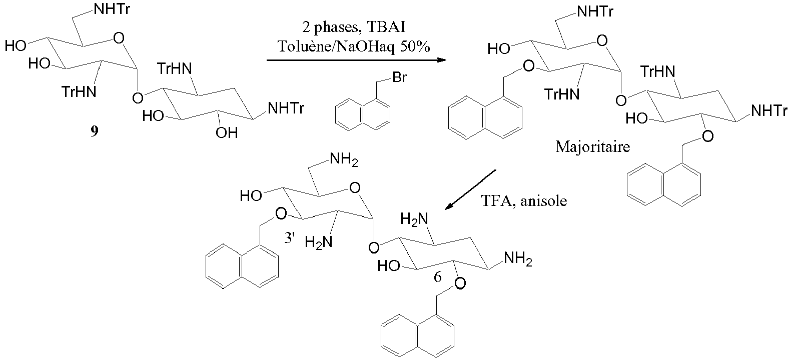

Selon un second aspect, l'alkylation se fait sélectivement en 6 (voie de droite sur le schéma ci-dessous), voire en 3'+4'+6 (voie de gauche sur le schéma ci-dessous), en fonction de la quantité d'halogénure d'alkyle (RX) en présence.

L'alkylation se fait par transfert de phase avec une phase aqueuse contenant de la soude (30 à 50%) et une phase organique composée de toluène (ou par exemple le dichlorométhane) et contenant le dérivé **9** dissous. L'agent de transfert de phase, en l'occurrence le fluorure (ou chlorure ou hydrogénophosphate...) de tétrabutylammonium (ou de tétraméthyl- ou triéthyl-ammonium etc ....) est ensuite ajouté puis l'halogénure d'alkyle, par exemple le chlorure de *para*-méthoxybenzyle (PMBCl).

Dans une première condition qui consiste à limiter la quantité d'halogénure d'alkyle (par exemple PMBCl) et la quantité d'agent de transfert de phase, il est possible de privilégier la formation du dérivé fonctionnalisé en position 6. Après isolement du dérivé 6 formé, celui peut être bisalkylé dans d'autres conditions en position 3' et 4' puis déprotégé en présence de TFA et d'anisole pour conduire au dérivé bisalkylé en position 3' et 4'.

Alternativement, il peut être trisalkylé dans des conditions différentes en position 3', 4' et 5 puis déprotégé en présence de TFA et d'anisole pour conduire au dérivé trisalkylé en position 3', 4' et 5.

Si le groupement introduit préalablement en position 6 est stable dans le mélange TFA/anisole, des dérivés 3',4',6 trialkylés mixtes (R≠ R') peuvent également être préparés après déprotection.

Dans une autre condition qui consiste à ne réaliser que la première étape d'alkylation par transfert de phase et à utilisant une quantité plus importante d'halogénure d'alkyle (par exemple PMBCl, 1NMBr, 2NMBr), il est possible de privilégier la formation du dérivé tris-alkylé en position 3'+4'+6.

Ces différentes voies sont représentées sur le schéma ci-dessous :

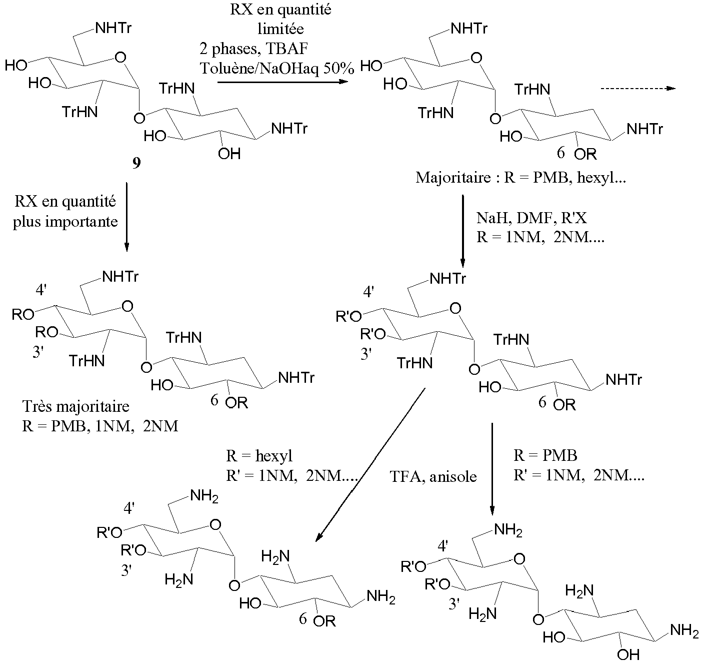

Ce schéma peut par exemple servir à la synthèse du :
- dérivé de la néamine *N-* tétratritylée 6-mono-2NM= 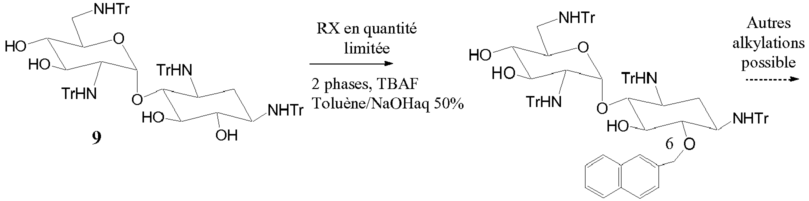
- dérivé 3',4',5-tri-2NM=

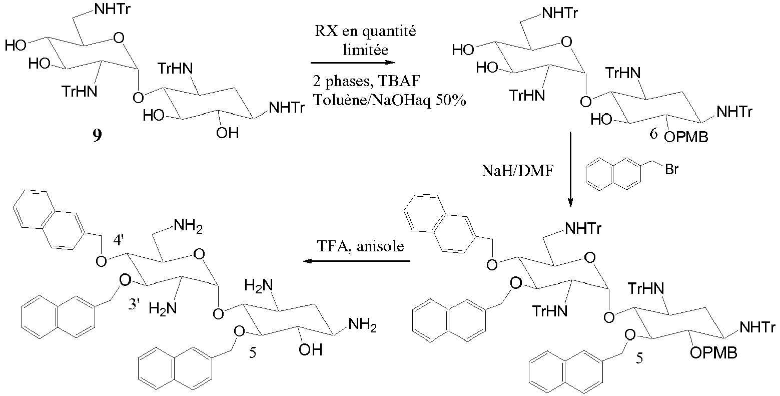

Ces différents schémas réactionnels ont été décrits plus avant en rapport avec des réactions d'alkylation. Le principe en reste toutefois le même pour la préparation des dérivés aryles, des dérivés esters, carbonates, carbamates, sulfonates ou sulfamates.

Pour ces dérivés, les réactions d'alkylation et/ou de protection décrites ci-dessus sont remplacées par des réactions d'arylation, d'acylation, de carbonatation, de carbamoylation, de sulfonylation, d'aminosulfonylation et/ou de silylation bien connues de l'homme du métier. A titre d'exemple, les réactions d'acylation sont réalisées dans le dichlorométhane avec un anhydride ou un chlorure d'acide, en présence d'une base telle que pyridine, *N,N-*diméthylaminopyridine, ou triéthylamine. Il est également envisageable de réaliser l'acylation enzymatiquement.

Par ailleurs, il apparaît que les groupements R ou R', voire R" mentionnés dans ces schémas réactionnels peuvent correspondre aux groupements R₂, R₃, R₄ et R₅, présents dans le composé final. Toutefois, la nature de ces groupements peut être modifiés et ce par deux mécanismes :
- les réactions d'alkylation (mais aussi d'arylation, d'acylation, de carbonatation, de carbamoylation, de sulfonylation ou d'aminosulfonylation) peuvent être ménagées et la nature de R modifiée entre chaque étape (entre les introductions aux différentes positions) ;
- le groupement porté en position 3' ou 4' ou 6 ou 5 peut être introduit en plusieurs étapes. Ainsi, après l'introduction de R ou R' à la position souhaitée, ce résidu peut être modifié par réactions successives.

### EXEMPLES DE REALISATION

L'invention et les avantages qui en découlent ressortiront mieux des exemples de réalisation suivants. Ceux-ci ne sont cependant en aucun cas limitatifs.

La présente invention va être illustrée plus avant à l'aide des composés dialkylés en position 3' et 6 ou trialkylés en position 3', 4' et 6, obtenus à l'issue de la synthèse selon la voie A telle que décrite ci-dessus et rappelée ici :

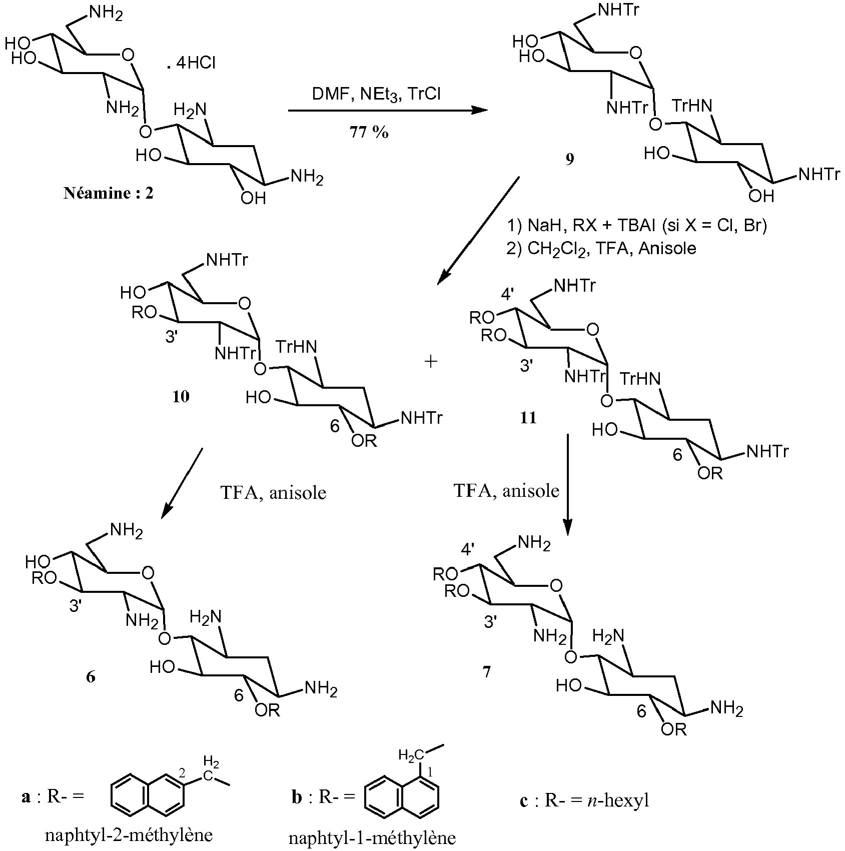

### PARTIE EXPERIMENTALE

### I - Synthèse des dérivés

### A/ Voie classique :

### 1/ Synthèse des dérivés 3',6-di(naphtyl-2-méthylène) néamine 6a et 3',4',6-tri(naphtyl-2-méthylène) néamine 7a

Le dérivé tritylé de la néamine **9** (2,0 g, 1,55 mmol) est mis en solution dans le DMF anhydre (20 mL), sous argon. On ajoute ensuite successivement l'hydrure de sodium (60 %, 217 mg, 5,42 mmol), l'iodure de tétrabutylammonium (572 mg, 1,55 mmol) et le 2-(bromométhyl)naphtalène (857 mg, 3.87 mmol). Le mélange est maintenu sous agitation pendant 24 h à température ambiante, puis 150 mL de dichlorométhane sont ajoutés. La phase organique est ensuite lavée avec 200 mL d'eau puis séchée sur sulfate de magnésium. Après évaporation des solvants sous pression réduite, le résidu obtenu est chromatographié sur gel d'alumine en utilisant un mélange cyclohexane/dichlorométhane : 50/50. Le produit **10a** (980 mg, 0,62 mmol, 40 %) et le produit **11a** (1,01 g, 0,59 mmol, 38 %) sont obtenus après séparation et évaporation des solvants sous pression réduite. Composé **10a** : SM (FAB⁺, NBA) m/z = 1594 [M + Na] ⁺, composé **11a** : SM (FAB⁺, NBA) m/z = 1735 [M + Na] ⁺

Le composé **10a** (602 mg, 0,39 mmol) et le composé **11a** (121 mg, 0,07 mmol) sont respectivement mis en solution dans un mélange de dichlorométhane et d'acide trifluoroacétique (v/v : 1/1) auquel une petite quantité d'anisole est ajoutée. Après 12 h de réaction à température ambiante, les solvants sont évaporés sous pression réduite et une extraction eau/diéthyléther est effectuée. Après concentration de la phase aqueuse sous pression réduite, le résidu est chromatographié sur colonne de phase inverse C18 avec un gradient d'élution eau/méthanol allant de 100/0 à 50/50. Les composés alors obtenus sont passés sur une colonne échangeuse d'ions en éluant à l'eau puis avec un mélange eau/ammoniaque (98/2). Après évaporation du solvant sous pression réduite, les composés sont repris dans une solution aqueuse de HCl 1N. Le composé **6a** et le composé **7a** sont obtenus sous forme de chlorhydrates après concentration et séchage.
**Composé 6a:** Rdt = 70 % ; RMN ¹H ( 400 MHz, D₂O) δ ppm = 7.84-7.89 (m, 8H, H-naphtyl), 7.47-7.50 (m, 6H, H-naphtyl), 5.86 (d, J = 3.6 Hz, 1H, H-1'), 5.05 (d, J = 12.0 Hz, 1H, CH₂-naphtyl), 5.02 (d, J = 12.0 Hz, 1H, CH₂-naphtyl), 4.88 (d, J = 12.0 Hz, 1H, CH₂-naphtyl), 4.85 (d, J = 12.0 Hz, 1H, CH₂-naphtyl), 4.04 (dd, J = 8.5 et 10.4 Hz , 1H, H-3'), 3.96 (m, 1H, H-5'), 3.91 (dd, J = 10.0 Hz, 1H, H-4), 3.82 (dd, J = 9.2 Hz, 1H, H-5), 3.65 (dd, J= 9.2 Hz, 1H, H-4'), 3.58 (dd, J= 9.2 Hz, 1H, H-6), 3.45 (dd, J= 3.6 et 10.4 Hz, 1H, H-2'), 3.43-3.28 (m, 3H, H-3, H-6'b, H-1), 3.22 (dd, J = 9.6 et 12.8 Hz, 1H, H-6'a), 2.42 (ddd, J = 4.0 et 12.4 Hz, 1H, H-2eq.), 2.01 (ddd, J = 12.4 Hz, 1H, H-2ax.) ; RMN ¹³C (100 MHz, D₂O) δ ppm = 132.9-134.6 (C-naphtyl), 126.3-128.6 (CH-naphtyl), 95.8 (C-1') 79.8 (C-6), 77.5 (C-4), 75.6 (C-5 et C-3'), 75.1 (CH₂-naphtyl), 70.9 (C-4'), 69.9 (C-5'), 52.6 (C-2'), 48.8 (C-1), 48.3 (C-3), 39.8 (C-6'), 28.1 (C-2) ; SM (DCI⁺) m/z = 603 [M + H] ⁺, 463, 303, 141; SMHR (ESI⁺) : [M+H]⁺ *m*/*z* théorique = 603.3183, trouvé = 603.3186, [M+Na]⁺ m/z théorique = 625.3002, trouvé = 625.3006.
**Composé 7a:** Rdt = 65 %, RMN ¹H (400 MHz, MeOD) δ ppm = 7.39-7.97 (m, 21H, H-naphtyl), 6.03 (d, J = 3.6 Hz, 1H, H-1'), 4.94-5.29 (m, 6H, CH₂-naphtyl), 3.57-3.59 (m, 2H, H-3', H-5'), 4.20 (dd, J= 9.6 Hz, 1H, H-4), 4.08 (dd, J= 9.2 Hz, 1H, H-5), 3.65-3.74 (m, 2H, H-6, H-4'), 3.49-3.56 (m, 2H, H-3, H-2'), 3.35-3.42 (m, 2H, H-6'b, H-1), 3.17 (dd, J= 9.6 et 13.2 Hz, 1H, H-6'a), 2.46 (ddd, J= 4.0 et 12.4 Hz, 1H, H-2eq.), 2.01 (ddd, J = 12.8 Hz, 1H, H-2ax.) ; RMN ¹³C (100 MHz, MeOD) δ ppm= 133.1-135.4 (C-naphtyl), 125.3-127.9 (CH-naphtyl), 95.2 (C-1'), 80.5 (C-6), 78.9 et 78.5 (C-4 et C-4'), 77.5 (C-3'), 76.3 (C-5), 74.7 et 74.4 (CH₂-naphtyl), 69.7 (C-5'), 53.4 (C-2'), 49.5 (C-1), 48.6 (C-3), 40.3 (C-6'), 29.5 (C-2) ; SM (DCI⁺) : *m*/*z* = 743 [M + H] ⁺, 603 [M + H - (naphtylméthylène)] ⁺, 441, 303, 141 ; SMHR (ESI⁺) : [M+H]⁺ *m*/*z* théorique = 743.3809, trouvé = 743.3810, [M+Na]⁺ m/z théorique = 765.3628, trouvé = 765.3628.

### 2/ Synthèse du dérivé 3',4',6-tri-n-hexyl néamine 7c

Le composé **7c** a été synthétisé selon le mode opératoire décrit pour les composés **6a** et **7a** en partant de 1,5 g de dérivé tritylé de la néamine **9**, de 1-iodohexane et sans ajout d'iodure de tétrabutylammonium. Le produit protégé intermédiaire pur **11c** est obtenu avec 50 % de rendement, après passage sur colonne d'alumine en utilisant comme éluant un mélange cyclohexane/dichlorométhane allant de 75/25 à 50/50 (SM MALDI, DHB, m/z = 1567 [M + Na]⁺). Après déprotection en milieu acide comme décrit précédemment, le produit **7c** est isolé par simple extraction eau/dichlorométhane et lavage à l'éther éthylique avec un rendement de 66 %. RMN ¹H (400 MHz, MeOD) δ ppm = 5.80 (d, J= 3.6 Hz, 1H, H-1'), 4.12 (ddd, J= 2.4 et 9.2 Hz, 1H, H-5'), 3.96 (ddd, J= 7.2 Hz, 1H de 1CH₂O), 3.54-3.89 (m, 8H, H-4, H-3', H-5, 5H de 3CH₂O), 3.11-3.39 (m, 7H, H-1, H-6, H-4', H-3, H-2', H-6'a, H-6'b), 2.38 (ddd, 1H, J= 4.0 et 12.0 Hz, H-2eq.), 1.87 (ddd, J = 12.0 Hz, 1H, H-2ax.), 1.55-1.75 (m, 6H, 3CH₂), 1.25-1.47 (m, 18H, 9CH₂), 0.90-0.96 (m, 9H, 3CH₃) ; RMN ¹³C (100 MHz, MeOD) δ ppm = 95.8 (C-1'), 81.1 (C-6), 79.6 (C-4 et C-4'), 77.2 (C-3'), 76.1 (C-5), 73.5, 73.3 et 73.0 (3CH₂O), 69.5 (C-5'), 53.4 (C-2'), 49.5 (C-1), 48.6 (C-3), 40.1 (C-6'), 31.5 (3CH₂), 29.9, 29.8 et 29.6 (C-2 et 3CH₂), 25.5, 25.4 et 25.2 (3CH₂), 22.3 (3CH₂), 13.0 (3CH₃) ; SM (MALDI, DHB) m/z = 575 [M + H]⁺.

### 3/ Synthèse du dérivé 3',4'-di-(naphtyl-2-méthylène) néamine 8a

Ce composé a été obtenu par déprotection en milieu acide du dérivé **11a.**
Le composé **11a** est mis en solution dans un mélange dichlorométhane-acide trifluoroacétique (v/v : 1/1) et de l'anisole est ajoutée. Après 24 h de réaction à température ambiante, les solvants sont évaporés sous pression réduite et une extraction eau/diéthyléther est effectuée. La purification menée ensuite est similaire à celle utilisée pour les autres dérivés de la néamine préparés. 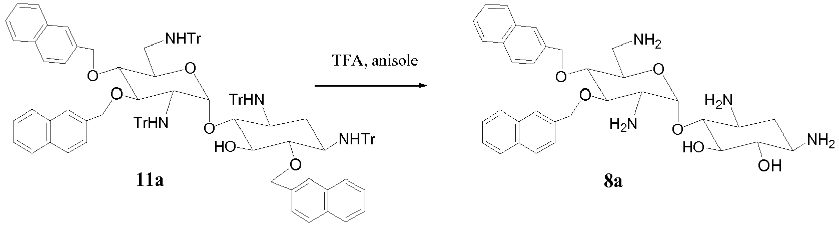 Rdt = 10 % (pour 2 étapes). RMN ¹H (400 MHz, D₂O) δ ppm = 7.10-7.90 (m, 14H, H-naphtyl), 5.87 (d, J= 3.6 Hz, 1H, H-1'), 4.87 (d, J= 12.0 Hz, 1H, CH₂-naphtyl), 4.78 (d, J = 12.0 Hz, 1H, CH₂-naphtyl), 4.62 (d, J = 12.0 Hz, 1H, CH₂-naphtyl), 4.59 (d, J = 12.0 Hz, 1H, CH₂-naphtyl), 4.05-4.20 (m, 2H, H-3', H-5'), 3.88 (dd, J = 10.0 Hz, 1H, H-4), 3.62 (dd, J= 9.6 Hz, 1H, H-5), 3.54-3.61 (m, 2H, H-4', H-2'), 3.40-3.51 (m, 2H, H-6, H-3), 3.19-3.29 (m, 2H, H-6'b, H-1), 3.13 (dd, J= 8.8 et 13.6 Hz, 1H, H-6'a), 2.42 (ddd, J= 4.0 et 12.4 Hz, 1H, H-2eq.), 1.79 (ddd, J= 12.0 Hz, 1H, H-2ax.) ; RMN¹³C (100 MHz, D₂O) δ ppm = 132.7-134.4 (C-naphtyl), 125.3-128.4 (CH-naphtyl), 95.4 (C-1'), 78.2 (C-4'), 77.7 (C-4), 75.7 (C-3'), 75.1 (C-5), 74.9 (CH₂-naphtyl), 72.3 (C-6), 69.7 (C-5'), 52.6 (C-2'), 49.6 (C-1), 48.4 (C-3), 39.8 (C-6'), 28.2 (C-2) ; SM (ESI⁺) m/z = 603 [M + H] ⁺, 463, 441, 301, 141.

### 4/ Synthèse du dérivé tétratritylé 6-(naphtyl-2-méthylène) néamine 18a

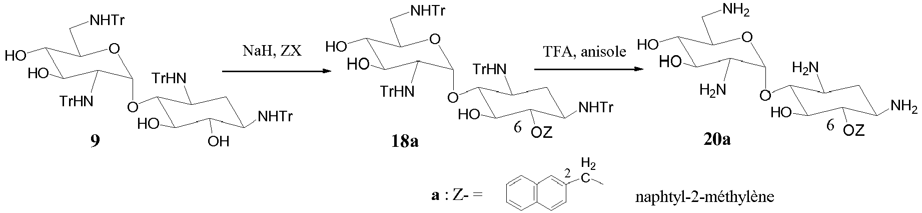

Le dérivé tritylé de la néamine **9** (502 mg) est dissout dans un mélange DMF/THF (3 ml/3 ml) à température ambiante sous argon puis NaH (170 mg ; 10 éq.) est ajouté. Après 30 min, le 2-(bromométhyl)naphtalène (130 mg ; 1,5 éq.) est additionné au mélange réactionnel qui est agité à température ambiante pendant 4 heures avant d'être concentré par évaporation sous pression réduite. Le résidu est dissout dans du dichlorométhane et lavé avec une solution saturée de chlorure d'ammonium, de l'eau puis de la saumure. La phase organique est séchée sur du sulfate de magnésium, filtrée puis évaporée à sec. Le produit brut est ensuite purifié sur colonne d'alumine avec comme éluant : CH₂Cl₂/ MeOH (100/0 puis 99,8/0,2) pour donner le dérivé **18a** (néamine tritylée et alkylée en position 6) avec un rendement de 12% (il reste essentiellement du composé de départ **9** qui n'a pas réagi et qui est récupéré) : SM (MALDI, DHB) m/z = 1454 [M + Na] ⁺, 1430 [M + H] ⁺, 1211 [M - Tr + Na] ⁺, 1187 [M - Tr + H] ⁺. Les groupements trityles peuvent être éliminés pour caractérisation par traitement avec de l'acide trifluoroacétique selon le mode opératoire décrit pour les autres dérivés de la néamine.
**Composé 20a** : Rdt = 82 % ; RMN ¹H ( 400 MHz, D₂O) δ ppm = 7.86-7.91 (m, 2H, H-naphtyl), 7.48-7.53 (m, 2H, H-naphtyl), 5.89 (d, J = 3.6 Hz, 1H, H-1'), 5.04 (d, J = 11.2 Hz, 1H, CH₂-naphtyl), 4.88 (d, J= 11.2 Hz, 1H, CH₂-naphtyl), 3.88-3.98 (m, 3H, H-3', H-5', H-4), 3.85 (dd, J= 8.8 et 9.2 Hz, 1H, H-5), 3.30-3.49 (m, 5H, H-3, H-4', H-2', H-6'b, H-1), 3.22 (dd, J = 6.8 et 13.6 Hz, 1H, H-6'a), 2.42 (ddd, J = 4.0 et 12.4 Hz, 1H, H-2eq.), 1.83 (ddd, J= 12.4 Hz, 1H, H-2ax.) ; RMN ¹³C (100 MHz, D₂O) δ ppm = 134.4 et 132.9 (C-naphtyl), 128.6, 128.0, 127.8, 127.6, 126.9 et 126.4 (CH-naphtyl), 96.0 (C-1'), 79.9 (C-6), 77.5 (C-4), 75.8 (C-5), 75.2 (CH₂-naphtyl), 70.6 (C-4'), 69.3 (C-5'), 68.2 (C-3'), 53.5 (C-2'), 48.9 (C-1), 48.4 (C-3), 40.1 (C-6'), 28.2 (C-2) ; SM (FAB⁺, NBA) : m/z = 463 [M + H] ⁺, 303, 161, 141; MSHR (ESI⁺) : [M+H]⁺ m/z théorique = 463.2557, trouvé = 463.2545.

Ce dérivé monoalkylé **20a** n'est pas intéressant en tant que tel mais la synthèse de son dérivé tritylé **18a**, donnée à titre d'exemple, constitue la première étape des voies B et C exposées ci-dessus.

### B/ Procédé biphasique en présence d'un agent de transfert :

### 1/ Synthèse du dérivé tétra-N-trityl 3'-O-para-methoxybenzyl de la néamine

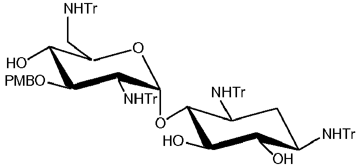

1 g de néamine tétra-*N-*tritylée **9** (1 eq, 0,775 mmol) est dissous dans 30 mL de toluène auquels sont successivement ajoutés 430 mg (1,5 eq) d'iodure de tétrabutylammonium et 0,15 mL de chlorure de *p*-méthoxybenzyle (PMBCl, 1,2 eq). 15 mL de soude (NaOH en solution aqueuse à 50 % m/m) sont additionnés à la solution, les deux phases ainsi obtenues sont mises sous une agitation vigoureuse pour donner une émulsion qui va tendre vers une couleur jaunâtre. Deux ajouts de 0,05 mL de PMBCl (0,4 eq) sont effectués après 24 h et 72 h. Après 4 jours, l'agitation est stoppée et le milieu réactionnel décanté, afin d'éliminer la soude. La phase organique est diluée par de l'acétate d'éthyle, lavée par une solution de chlorure d'ammonium saturée et séchée sur sulfate de magnésium anhydre. Le solvant est éliminé par évaporation sous pression réduite. La structure est confirmée en dérivant le composé majoritaire par réaction dans le DMF avec l'hydrure de sodium et l'iodométhane en excès. L'élimination des groupements protecteurs se fait dans le dichlorométhane par action d'acide trifluoroacétique (TFA) et de quelques gouttes d'anisole sur le mélange brut obtenu précédemment.
RMN ¹H (MeOD, ppm, 400 MHz): 5,68 (s, H-1) ; 4,10 (m, H-5') ; 4,02 (t, J = 9,4 Hz, H-4), 3,95 (t, J = 7,6 Hz, H-3') ; 3,73 (t, J = 9.4 Hz, H-6) ; 3,69-3,29 (m, H-2', H-5, H-3, H-6', H-4', H-6', H-1) ; 3,66 ; 3,60 ; 3,54 (3 s, H-OMe) ; 2,48 (m, H-2 eq) et 1,87 (m, H-2 ax).
RMN ¹³C (MeOD, ppm, 100 MHz): 164 (CO TFA) ; 117 (CF₃ TFA) ; 94,40 (C-1') ; 84,99 (C-5) ; 79,50 (C-4') ; 77,50 (C-3') ; 76,23 (C-4) ; 73,40 (C-6) ; 71,51 (C-5') ; 60,88 ; 60,84 ; 60,43 (3 C-OMe) ; 52,47 (C-2') ; 50,70 (C-1) ; 49,63 (C-3) ; 40,30 (C-6') et 29.00 (C-2).

### 2/ Synthèse du dérivé 4',6-di-O-(3-[(2-naphtyl)méthylène] de la néamine

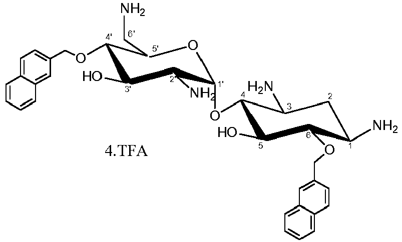

Sous atmosphère inerte, 33 mg (3 eq, 0,83 mmol) d'hydrure de sodium (60 % en suspension dans d'huile) sont ajoutés à une solution de 390 mg de néamine-tetra-*N-*Tr-3'-*O*-PMB (1 eq, 0,28 mmol) dans 15 mL de DMF. Le réacteur est mis sous agitation et est refroidi par un bain de glace. Après 15 min, 152 mg (2,5 eq, 0,69 mmol) de 2-(bromomethyl)naphtalène sont ajoutés, le bain à 0°C est maintenu pendant 30 min puis le mélange réactionnel est agité à température ambiante durant 12 h. Le suivi de la réaction se fait par CCM (toluène/acétate d'éthyle) ; 9/1). Le solvant du milieu réactionnel est éliminé par évaporation sous pression réduite. Le brut ainsi obtenu est dissous par de l'acétate d'éthyle puis lavé deux fois par de l'eau distillée et une solution saturée en NaCl. La phase aqueuse est séchée sur sulfate de magnésium anhydre puis concentrée sous pression réduite. Le produit majoritaire est directement déprotégé à 0°C dans 10 mL de dichlorométhane par action de 5 mL de TFA et de quelques gouttes d'anisole. Après 2 h d'agitation, le TFA est éliminé par évaporation et co-évaporation sous pression réduite avec de l'éthanol puis du toluène. Le dérivé est alors purifié par chromatographie sur gel de silice greffée C₁₈ (eau 100% jusqu'à eau/méthanol 70/30), pour donner 40 mg d'une gomme translucide. Rendement : 40 %
RMN ¹H (400 MHz, D₂O) δ ppm = 7,90-7,50 (m, 14H, H-naphtyl) ; 6,01 (d, *J* = 3,6 Hz, 1H, H-1') ; 5,20 (m, 2H, CH₂-naphtyl) ; 4,90 (m, 2H, CH₂-naphtyl) ; 4,27 (dd, *J* = 8 et 12 Hz, 1H, H-3') ; 4,18 (m, 1H, H-5') ; 4,11 (dd, *J* = 10 Hz,1H, H-4) ; 3,91 (dd, *J* = 10 Hz, 1H, H-5) ; 3,64 (dd, *J* = 8 Hz, 1H, H-6) ; 3,28-3,46 (m, 5H, H-1, H-3, H-2', H-4', H-6'b) ; 2,98 (dd, *J* = 8 et 12 Hz, 1H, H-6'a) ; 2,50 (m, 1H, H-2b) ; 2,08 (m, 1H, H-2a); RMN ¹³C (100 MHz, D₂O) δ ppm = 132,0-135,0 (6C-naphtyl) ; 129,9-127,3 (14CH-naphtyl) ; 97,2 (C-1') ; 82,0 (C-6) ; 80,5 (C-4') ; 79,5 (C-4) ; 78,0 (C-5) ; 76,4 (CH₂-naphtyl) ; 76,1 (CH₂-naphtyl) ; 70,6 (C-3') ; 70,3 (C-5') ; 55,6 (C-2') ; 50,8 (C-1) ; 50,2 (C-3) ; 42,1 (C-6') ; 30.0 (C-2); HRMS (ESI⁺): [M+H]⁺ m/z calculé 603.3183, trouvé 603.3199, [M+Na]⁺ m/z calculé 625.3002, trouvé 625.3005, [M+K]⁺ m/z calculé 641.2741, trouvé 641.2726.

### 3/ Synthèse du dérivé tétra-N-trityl de la paromamine

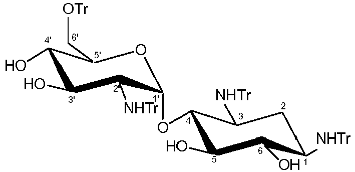

Dans un réacteur placé sous atmosphère inerte, 2,5 g (1 eq, 5,78 mmol) de paromamine, 4HCl sont mis en suspension dans 100 mL de DMF anhydre et 5 ml de triéthylamine (stockée sur hydroxyde de potassium). La solution est mise sous agitation et après 1 h, 8 g (5 eq, 0,029 mol) préalablement dissous dans du DMF anhydre sont ajoutés goutte à goutte à la suspension de paromamine. Après 12 h, une dizaine de mg de 4,4'-*N-*diméthylaminopyridine est ajoutée au milieu réactionnel. Au bout de 24 h, le solvant est évaporé sous pression réduite. Le résidu obtenu est dissous dans du dichlorométhane puis lavé deux fois avec de l'eau distillée et une solution aqueuse saturée en NaCl. Le composé est purifié par chromatographie sur gel de silice traité par de la triéthylamine (cyclohexane/AcOEt) pour obtenir une poudre blanche (3,32 g, 77%).
LRMS (MALDI, DHB) m/z =1331 [M + K]⁺, 1315 [M+Na]⁺, 1293 [M+H]⁺

### 4/ Synthèse du dérivé 3',6-di-O-[(2-naphtyl)méthylène] de la paromamine 6a

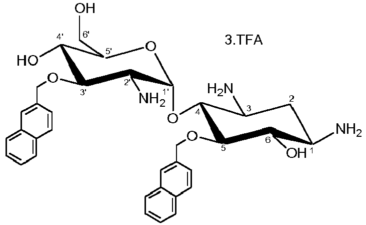

1 g (1 eq, 0,774 mmol) de paromamine *N*-tétratritylée est dissous dans 30 mL de toluène. 750 mg (3 eq, 2,32 mmol) de bromure de tétrabutylammonium puis 510 mg (3 eq, 2,32 mmol) de 2-(bromométhyl)naphtalène sont ajouté à la solution. 15 mL d'une solution de soude à 50 % (m/m) sont additionnés au mélange. Les deux phases ainsi obtenues sont soumises à une agitation soutenue pour donner une émulsion qui devient jaune au cours du temps. Après 24 h, le milieu réactionnel est mis à décanter, la soude est éliminée, puis la phase organique est diluée avec du toluène. On lave celle-ci deux fois avec de l'eau distillée puis avec une solution aqueuse saturée en NaCl, pour être ensuite séchée sur sulfate de magnésium anhydre. Le solvant est éliminé par évaporation sous pression réduite pour donner un résidu de coloration brunâtre. Le composé attendu est alors purifié par chromatographie sur alumine basique *via* un gradient d'élution (cyclohexane/toluène ; toluène/acétate d'éthyle) pour donner une poudre jaunâtre (382 mg, 31,4 %). 300 mg de paromamine *N*-tétratritylée 3',6-di-*O*-[(2-naphtyl)méthylène] sont déprotégés à 0°C dans 10 mL de dichlorométhane par action de 5 mL de TFA et de quelques gouttes d'anisole. Après 2 h d'agitation, le TFA est éliminé par évaporation sous pression réduite par co-distillation avec de l'éthanol puis du toluène. Le dérivé est alors purifié par chromatographie sur gel de silice greffée C₁₈ (Eau 100% jusqu'à Eau/méthanol 70/30) pour donner une gomme translucide (62 mg, 30 %).
RMN ¹H (400 MHz, D₂O) δ ppm = 7,85-7,55 (m, 14H, H-naphtyl) ; 5,65 (d, 1H, H-1') ; 5,10 (m, 2H, CH₂-naphtyl) ; 4,95 (m, 2H, CH₂-naphtyl) ; 4,06 (dd, 1H, H-3') ; 3,9 (m, 4H, H-4', H-5, H-5', H-6'ₐ) ; 3,75 (m, 3H, H-4', H-6, H-6'ₐ) ; 3,55 (m,2H, H-3, H-2') ; 3,40 (dd, 1H, H-1) ; 2,50 (m, 1H, H-2b) ; 1,85 (m, 1H, H-2a); RMN ¹³C (100 MHz, D₂O) δ ppm = 167,8 et 164,4 (CO TFA) ; 136,3-134,5 (6C-naphtyl) ; 130,29-128,0 (14CH-naphtyl) ; 98,4 (C-1'); 82,0 (C-6) ; 82,2 (C-4) ; 81,2 (C-6) ; 78,1 (C-3') ; 76,8 (CH₂-naphtyl) ; 76,7 (CH₂-naphtyl) ; 76,0 (C-5') ; 71,43 (C-4') ; 61,7 (C-6') ; 54,5 (C-2') ; 50,4 (C-3, C-1) ; 29,9 (C-2)

### 5/ Synthèse du dérivé 3',6-di-O-[(1-naphtyl)méthylène] de la néamine 6b

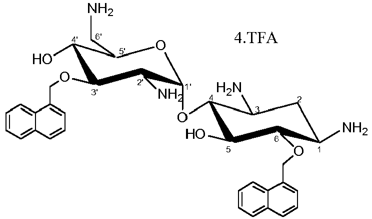

1 g de néamine *N*-tétratritylée **9** (1 eq, 0,775 mmol) est dissous dans 30 mL de toluène puis sont successivement ajoutés 428 mg (1,5 eq) d'iodure de tétrabutylammonium et 350 µL (3 eq.) de 1-(chlorométhyl)naphtalène. 15 mL de soude à 50 % (m/m) sont additionnés à la solution, les deux phases ainsi obtenues sont mises sous une agitation vigoureuse pour donner une émulsion qui va tendre vers une couleur jaunâtre. Le suivi de la réaction se fait par CCM (toluène/acétate d'éthyle ; 9/1). Trois ajouts de 175 µL de 1-(chlorométhyl)naphtalène (1,5 eq) sont effectués après 12, 24 et 48 h. Après 7 jours, l'agitation est stoppée et le milieu réactionnel décanté, afin d'éliminer la soude. La phase organique est diluée par du toluène, lavée par une solution de chlorure d'ammonium saturée et séchée sur sulfate de magnésium anhydre. Le solvant est éliminé par évaporation sous pression réduite. Le composé attendu est alors purifié par chromatographie sur alumine *via* un gradient d'élution (cyclohexane/toluène ; toluène/acétate d'éthyle) pour donner une poudre jaunâtre (500 mg, 41 %). 300 mg du composé isolé sont déprotégés à 0°C dans 10 mL de dichlorométhane par action de 5 mL de TFA et de quelques gouttes d'anisole. Après 2 h d'agitation, le TFA est éliminé par évaporation et co-évaporation sous pression réduite avec de l'éthanol puis du toluène. Le dérivé attendu est alors purifié par chromatographie sur gel de silice greffée C₆H₅ (eau 100% jusqu'à eau/méthanol 70/30), pour donner une gomme translucide (115 mg, 60,5 %). LRMS (MALDI, DHB) m/z = 641 [M + K]⁺, 629 [M+Na]⁺, 604 [M+H]⁺.

### 6/ Synthèse du dérivé tétra-N-tritylé 6-O[2-naphtyl)méthylène] de la néamine

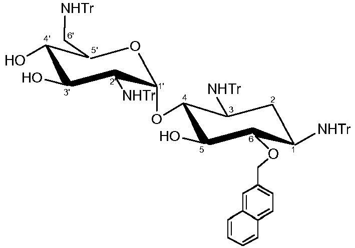

2 g de néamine *N*-tétratritylée **9** (1 eq, 1,55 mmol) sont dissous dans 60 mL de toluène puis sont successivement ajoutés 486 mg (1,5 eq) de fluorure de tétrabutylammonium hydraté et 411 mg de 2-(bromométhyl)naphtalène (1,2 eq). 30 mL de soude à 50 % (m/m) sont additionnés à la solution, les deux phases ainsi obtenues sont mises sous une agitation vigoureuse pour donner une émulsion qui va tendre vers une couleur jaunâtre. Après 12 h de réaction, le milieu réactionnel est mis à décanter, la soude est éliminée, puis la phase organique est diluée avec du toluène. On lave celle-ci deux fois avec de l'eau distillée puis avec une solution aqueuse saturée en NaCl. La phase organique est ensuite séchée sur sulfate de magnésium anhydre. Après évaporation sous pression réduite, le composé attendu est purifié par chromatographie sur alumine *via* un gradient d'élution (cyclohexane/toluène ; toluène/acétate d'éthyle) pour donner une poudre jaunâtre (450 mg, 18,5 %). LRMS (MALDI, DHB) m/z = 1454 [M+Na]⁺ , 1430 [M+H]⁺, 1211 [M-Tr+Na]⁺, 1187 [M-Tr+H]⁺.

### 7/ Synthèse du dérivé tétra-N-trityl 6-O-PMB de la néamine

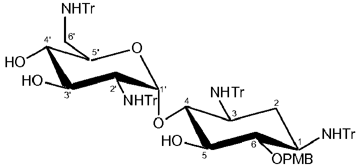

1 g de néamine tétra-*N*-tritylée **9** (1 eq, 0,775 mmol) est dissous dans 30 mL de toluène puis sont successivement ajoutés 243 mg (1 eq) de fluorure de tétrabutylammonium hydraté et 0,31 mL (2,5 eq) de PMBCl. 15 mL de soude à 50 % (m/m) sont additionnés à la solution, les deux phases ainsi obtenues sont mises sous une agitation vigoureuse pour donner une émulsion qui va tendre vers une couleur jaunâtre. Après 2 h, l'agitation est stoppée et le milieu réactionnel décanté, afin d'éliminer la soude. La phase organique est diluée avec de l'acétate d'éthyle, lavée par une solution aqueuse saturée de chlorure d'ammonium et séchée sur sulfate de magnésium anhydre. Le solvant est éliminé par évaporation sous pression réduite. La structure est confirmée en dérivant le composé majoritaire par réaction dans le DMF avec l'hydrure de sodium (60 % dans l'huile) et l'iodométhane en excès. La déprotection se fait dans le dichlorométhane par action de TFA et de quelques gouttes d'anisole sur le résidu d'évaporation obtenu précédemment.
RMN ¹H (MeOD, ppm, 400 MHz): 5,68 (s, H-1) ; 4,10 (m, H-5') ; 4,02 (t, J = 9,4 Hz, H-4) ; 3,95 (t, J = 7,6 Hz, H-3') ; 3,73 (t, J = 9,4 Hz, H-6) ; 3,69-3,29 (massif, H-2', 5, 3, 6', 4', 6', 1) ; 3,66 ; 3,60 ; 3,54 (3 s, H-OMe) ; 2,48 (m, H-2 eq) et 1,87 (m, H-2 ax). RMN ¹³C (MeOD, ppm, 100 MHz): 164 (CO TFA) ; 117 (CF₃ TFA) ; 94,40 (C-1') ; 84,99 (C-5) ; 79,50 (C-4') ; 77,50 (C-3') ; 76,23 (C-4) ; 73,40 (C-6) ; 71,51 (C-5'); 60,88 ; 60.84 ; 60,43 (3 C-OMe) ; 52,47 (C-2') ; 50,70 (C-1) ; 49,63 (C-3) ; 40,30 (C-6') et 29,00 (C-2).

### 8/ Synthèse du dérivé 3',4',5-tri-O-[(2-naphtyl)méthylène] de la néamine

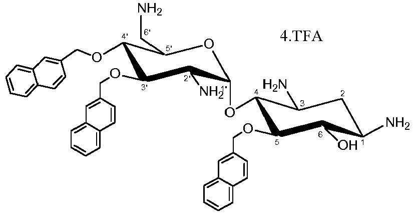

Sous atmosphère inerte, 172 mg (10 eq, 4,3 mmol) d'hydrure de sodium (60 % en suspension dans de l'huile) sont ajoutés à une solution de 600 mg de néamine tétra-*N-*tritylée 6'-*O*-PMB (1 eq, 0,43 mmol) dans 15 mL de DMF anhydre. Le réacteur est mis sous agitation et est refroidi avec un bain de glace. Après 15 min, 569 mg (6 eq, 2,58 mmol) de 2-(bromométhyl)naphtalène (préalablement dissous dans 5 mL de DMF anhydre) sont ajoutés goutte à goutte, le bain à 0°C est conservé pendant 30 min puis le mélange réactionnel est agité à température ambiante durant 48 h. Le suivi de la réaction se fait par CCM (toluène 100 %). Le solvant est éliminé par évaporation sous pression réduite. Le résidu est dissous dans le toluène puis lavé deux fois avec de l'eau distillée et une solution aqueuse saturée en NaCl. La phase aqueuse est séchée sur sulfate de magnésium anhydre puis concentrée sous pression réduite. Le composé attendu est alors purifié par chromatographie sur alumine basique *via* un gradient (cyclohexane 100 % à cyclohexane/toluène 40/60) pour donner une poudre jaunâtre (300 mg, 38 %). 300 mg du composé isolé sont déprotégés à 0°C dans 10 mL de dichlorométhane par action de 5 mL de TFA et de quelques gouttes d'anisole. Après 2 heures d'agitation, le TFA est éliminé par évaporation sous pression réduite en générant un azéotrope avec de l'éthanol puis du toluène. Le dérivé attendu est alors purifié par chromatographie sur gel de silice greffée C₁₈ (eau 100% jusqu'à eau/méthanol 70/30), pour donner une gomme translucide (120 mg, 64,5 %).
RMN ¹H (400 MHz, D₂O) δ ppm = 7,9-7,3 (m, 21H, H-naphtyl) ; 5,60 (d, 1H, H-1') ; 5,30 (m, 2H, CH₂-naphtyl) ; 5,0 (m, 2H, CH₂-naphtyl) ; 4,7 (m, 2H, CH₂-naphtyl) ; 4,55 (dd, 1H, H-3') ;4,25 (m, 1H, H-3') ; 4,10 (m, 1H, H-4) ; 3,65 (m, 2H, H-5, H-6) ; 3,5 (m, 4H, H-2', H-4', H-3, H-6'_{b}) ; 3,30 (dd, 1H, H-1) ; 3,00 (m, 1H, H-6'ₐ) ; 2,50 (m, 1H, H-2_{b}) ; 2,25 (m, 1H, H-2ₐ); RMN ¹³C (100 MHz, D₂O) δ ppm = 163,6 et 164,4 (CO TFA) ; 137,1-134,6 (9C-naphtyl) ; 129,6-126,6 (21CH-naphtyl) ; 93,5 (C-1') ; 85,0 (C-5') ; 79,8 (C-4) ; 77,1 (CH₂-naphtyl) ; 75,9 (C-3') ; 74,8 (C-6) ; 74,5 (C-5) ; 74,2 (CH₂-naphtyl) ; 73,9 (C-4') ; 73,7 (CH₂-naphtyl) ; 51,6-50,33 (C-3, C-1, C-2') ; 39,7 (C-6') ; 29,2 (C-2). LRMS (MALDI, DHB) m/z =782 [M + K]⁺, 766 [M+Na]⁺, 744 [M+H]⁺.

### II - Activité antibactérienne

### 1/ Molécules testées:

Les molécules sont mises en solution dans l'eau ou le DMSO à une concentration de 5 ou 10 mg/ml, puis les solutions sont diluées si nécessaire. Pour chacune d'elle un volume de 200 µl est disposé en plaque de 96 puits.

### 2/ Souches testées:

Les souches suivantes servent au test de l'activité antimicrobienne :
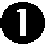 *Escherichia coli* ATCC25922 = souche gram négatif, sensible.
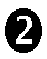 *Staphylococcus aureus* ATCC25923 = gram +, souche clinique sensible.
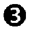 *Staphylococcus aureus* 1199B = gram +, souche clinique mutante surexprimant une pompe d'efflux NorA de la famille MFS, résistante aux fluoroquinolones, dont la ciprofloxacine.
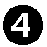 *Staphylococcus aureus* RN4220/pUL5054 = gram +, souche surexprimant len transporteur ABC MrsA, contenant le plasmide multicopies pUL5054 porteur du gène *msr(A)* : EryR.
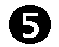 *Staphylococcus aureus* APH2"-AAC6' = gram +, souche résistante de type MRSA (« methicillin résistant staphylococcus aureus ») capable de modification enzymatique des aminosides : résistance enzymatique par action de l'aminoglycoside-6'-*N-*acétyltransférase/2"-*O*-phosphoryltransférase.
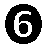 *Staphylococcus aureus* APH3' = gram +, souche résistante de type MRSA (« methicillin resistant staphylococcus aureus ») capable de modification enzymatique des aminosides : résistance enzymatique par action de l'aminoglycoside-3'-*O*-phosphoryltransférase.
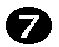 *Staphylococcus aureus* ANT4' = gram +, souche résistante de type MRSA (« methicillin resistant staphylococcus aureus ») capable de modification enzymatique des aminosides : résistance enzymatique par action de l'aminoglycoside-4'-*O*-(3-phosphoryltransférase.

### 3/ Tests microbiologigues:

### A- Validation des souches

Avant d'être utilisée, chaque souche est testée individuellement quant à son profil de résistance antibiotique. Cela signifie que pour chaque souche, la CMI (concentration minimale inhibitrice) d'un ou plusieurs antibiotiques de référence est testée, afin de s'assurer de la stabilité phénotypique de la bactérie.

Les antibiotiques suivants ont été testés :
*Escherichia coli* ATCC25922 (souche 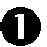 ) : Ampicilline, kanamycine, tétracycline, ciprofloxacine.
*Staphylococcus aureus* ATCC25923 (souche 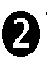 ) : Ampicilline, kanamycine, tétracycline, ciprofloxacine.
*Staphylococcus aureus* 1199B (souche 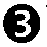 ) : Ciprofloxacine, norfloxacine, péfloxacine.
*Staphylococcus aureus* RN4220/pUL5054(souche 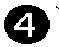 ) : Erythromycine.
*Staphylococcus aureus* APH2"-AAC6', APH3' et ANT4' (souches 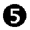 , 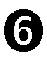 et 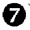 ) : Kanamycine, tobramycine, amikacine, gentamicine.

Dans un premier temps, les souches sont sorties du stock conservé à -80°C, et repiquées sur des boites de Pétri contenant du milieu MH gélosé. Ces boites sont incubées toute la nuit à 37°C, puis soit utilisées dès le lendemain, soit conservées à 4°C. Une boite peut ainsi être utilisée durant 2-3 semaines.

La veille du test, une préculture de 1 mL de milieu MH est ensemencée à partir d'une boite gélosée, et mise à incuber toute la nuit à 37°C.

Le lendemain, les tests sont effectués en plaques 96 puits, à l'aide du robot Biomek2000 (Beckman). Les solutions d'antibiotiques y sont diluées d'un facteur de 2 en 2 et réparties dans les puits de manière à couvrir une large gamme de concentrations, dans laquelle doit se trouver la valeur attendue. Un volume d'inoculum constitué de la préculture de la veille diluée au 1/100^{ème} est alors ajouté à chacun des puits. Chaque puits est doublé. Un témoin de non contamination (blanc), constitué de milieu de culture uniquement, et un témoin de croissance, constitué d'inoculum et de milieu de culture sans antibiotique, sont également prévus.

La plaque est mise à incuber à 37°C. Les mesures de prolifération bactérienne se font par lecture de la densité optique à 620 nm (DO₆₂₀ₙₘ) après 17-18 heures d'incubation, et les valeurs obtenues sont comparées avec celles trouvées dans la littérature. Si les valeurs correspondent (à une dilution près), la souche est dite « validée ».

### B- Criblage

Les tests se déroulent là aussi dans des plaques de 96 puits, à l'aide du robot Biomek2000 (Beckman). Chaque test est réalisé 2 fois. Un test consiste à mettre en présence la molécule à tester avec la souche bactérienne. Ainsi, la préculture bactérienne de la veille est diluée au 1/100^{ème}, et l'inoculum obtenu est additionné de la molécule à une concentration finale de 100 µg/ml (valeur choisie arbitrairement). La plaque est mise à incuber à 37°C, et la cinétique de prolifération bactérienne est suivie sur 24 heures, avec des mesures de DO₆₂₀ₙₘ à 0, 1, 4, 7, et 24 heures. Sur chaque plaque figurent également des témoins de croissance, de non-contamination, et de résistance/sensibilité à un antibiotique de référence.

Pour chaque puits, un score est attribué :
Score 1000 = la molécule n'a pas d'effet sur la prolifération bactérienne au regard du témoin de croissance.
Score 100 = après 24 heures d'incubation, la prolifération bactérienne est moindre au regard du témoin de croissance : environ 10% seulement de la valeur du témoin.
Score 10 = après 24 heures d'incubation, la prolifération bactérienne est quasi-nulle au regard du témoin de croissance : moins de 10% de la valeur du témoin.

### C- Détermination de la CMI

Les molécules donnant un score de 10 ou 100 au criblage sont sélectionnées afin de déterminer leur CMI vis-à-vis des souches testées.

Comme dans le cadre de la validation des souches, la solution de la molécule sélectionnée est diluée d'un facteur de 2 en 2 au moyen du robot Biomek2000 dans des plaques de 96 puits de manière à couvrir une large gamme de concentration. A chacun de ces puits est ajouté un volume d'inoculum constitué de la préculture de la veille diluée au 1/100^{ème}. Chaque puits est doublé. Là encore, des témoins de non contamination, de croissance, et de résistance/sensibilité à un antibiotique de référence sont intégrés à l'expérience. La plaque est mise à incuber à 37°C, et la cinétique de prolifération bactérienne est suivie sur 24 heures, avec des mesures de DO₆₂₀ₙₘ à 0, 1, 4, 7, et 24 heures.

La CMI est la concentration la plus faible trouvée capable d'inhiber la prolifération bactérienne après 24 heures d'incubation. N'ayant pas d'idée a priori de cette valeur, il est possible que celle-ci ne se trouve pas dans la gamme de dilution choisie. Il est alors nécessaire de réitérer l'opération en ajustant cette gamme.

### RESULTATS

Les effets antibiotiques ont donc été mesurés en termes de concentrations minimales inhibitrices de la croissance bactérienne (CMI) sur différentes bactéries Gram négatif et Gram positif, résistantes ou non.

### 1/ Concernant les bactéries gram(+) :

Les résultats de CMI, exprimées en microgrammes/mL, vis-à-vis de sont présentés dans le tableau I ci-dessous pour un certain nombre de molécules dans le tableau suivant et sont à comparer avec celles de la néamine et de la néomycine :

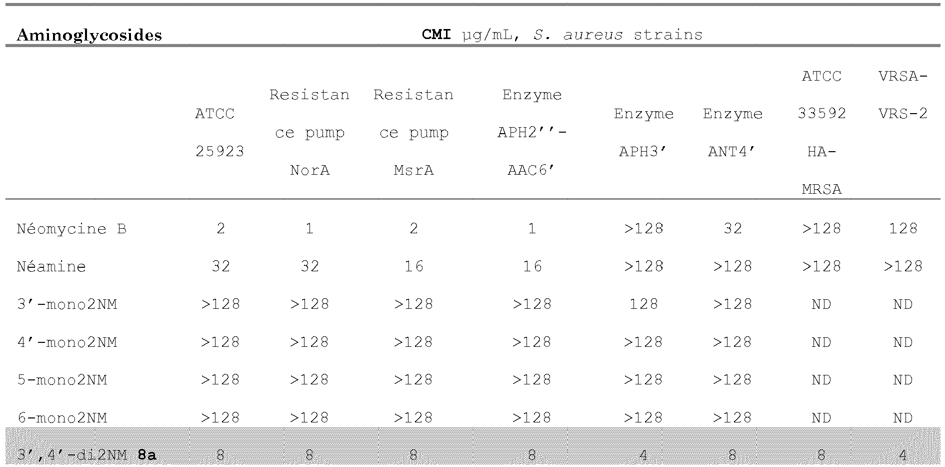

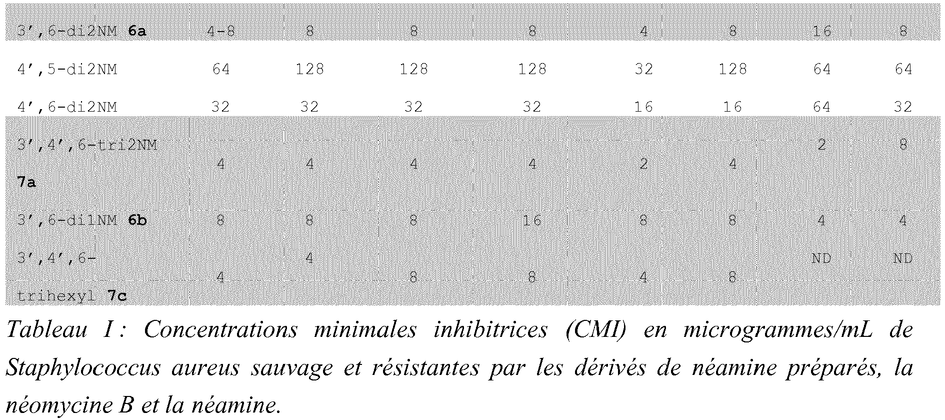

On constate que des dérivés difonctionnalisés ou trifonctionnalisés révèlent des effets antibiotiques très intéressants, en particulier sur des bactéries résistantes aux aminoglycosides classiques.

### 2/ Concernant les bactéries gram(-) :

Des mesures similaires ont été réalisées sir différentes bactéries gram (-). Les résultats correspondant sont présentés dans les tableaux II et III ci-dessous. Les souches testées ont été mises à disposition par R. Vanhoof (*E. coli* PAZ505H8101 and L58058.1, P. *aeruginosa* ATCC 27853, Psa.F03, *A. lwoffi* Al.88-483), Y. Glupczynski '*C*. *amalonaticus* Ca06AB0010), J.C. Pechere (*P. aeruginosa* PA02, PA03), P. Plésiat (*P*. *aeruginosa* PA01, PA21, PA22), H. Schweizer (*P. aeruginosa* PA405, PA406).

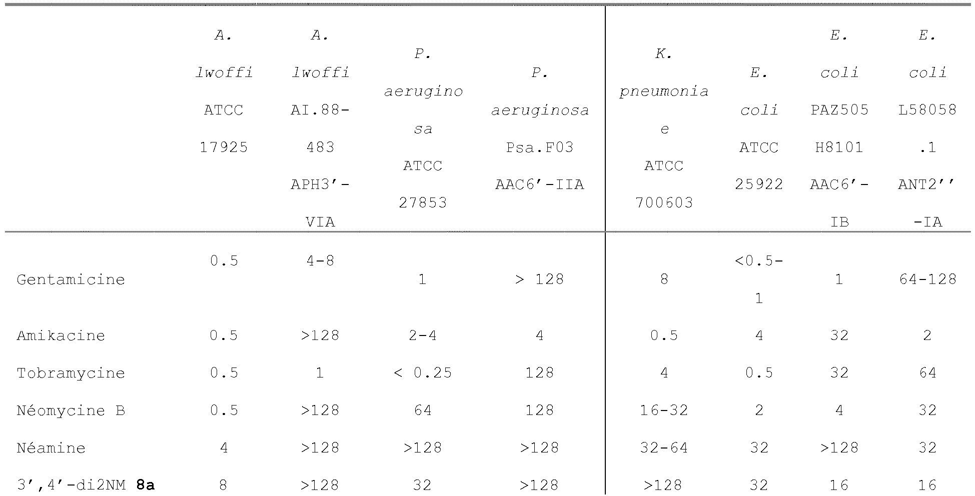

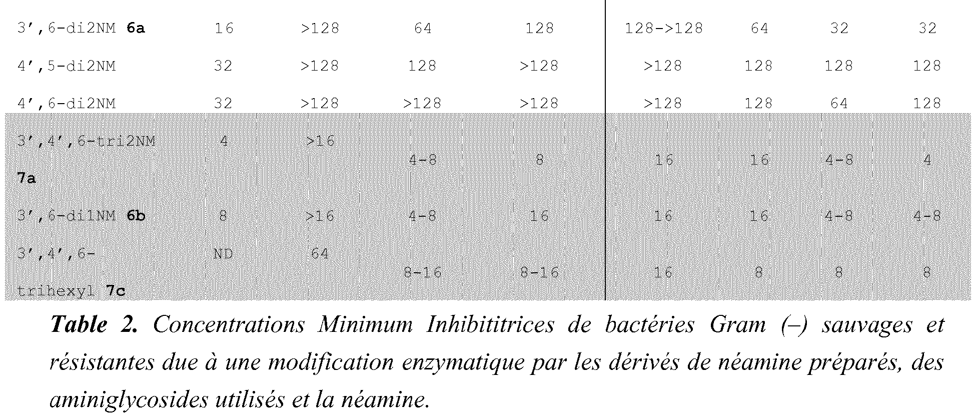

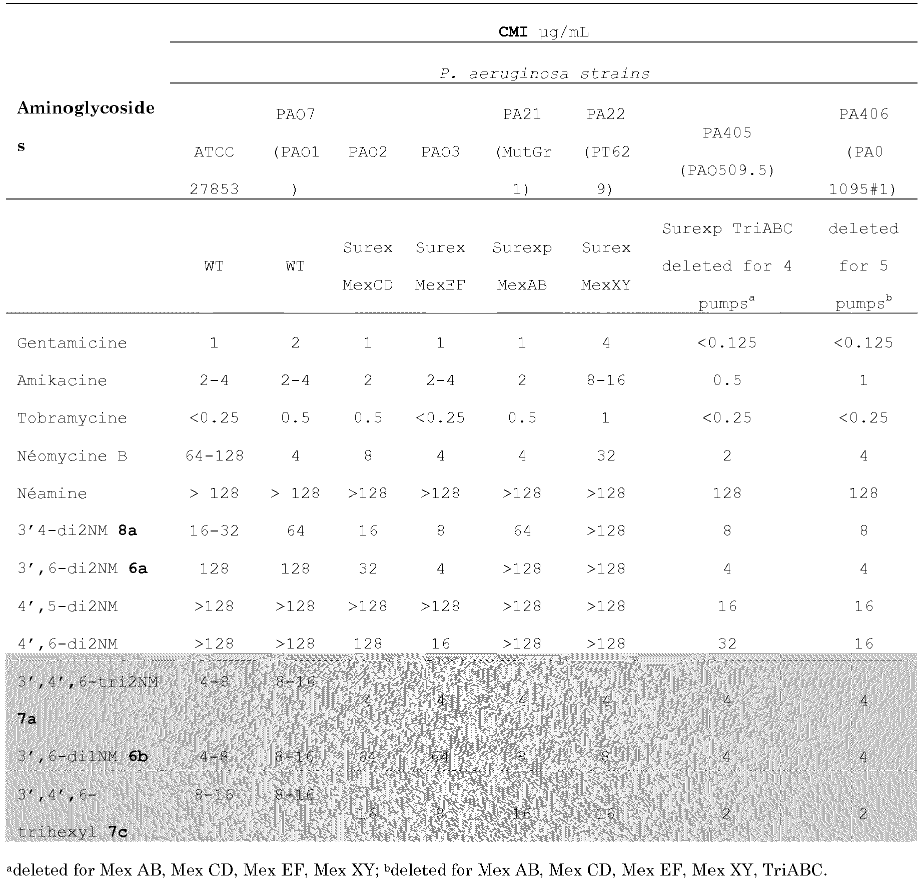

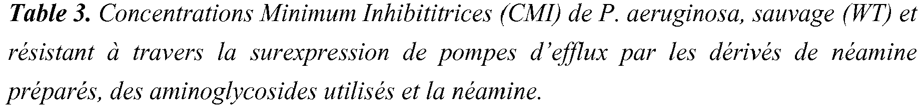

On constate que les dérivés 3',6-di1NM et 3',4',6-tri2NM (7) sont aussi actifs sur des bactéries résistantes exprimant une enzyme de méthylation de l'ARN (r-methylase) : C. *amalonaticus* arm 06AB0010, *E. coli* 06AB003 arm, *E. aerogenes* 06AB008 arm (CMI = 4-16 µg/mL). Au contraire, ces bactéries sont totalement résistantes à la gentamicine, l'amikacine et la tobramycine (CMI > 128 µg/ml).

### REFERENCES

D. Moazed, H. F. Noller. "Interaction of antibiotics with functional sites in 16S ribosomal RNA". Nature 1987, 327, 389-394.
E. Riguet, S. Tripathi, B. Chaubey, J. Désiré, V. N. Pandey, J.-L. Décout. "A peptide nucleic acid-neamine conjugate that targets and cleaves HIV-1 TAR RNA inhibits viral replication". J. Med. Chem. 2004, 47, 4806-4809.
E. Riguet, J. Désiré, C. Bailly, J.-L. Décout. "A Route For Preparing New Neamine Derivatives Targeting Hiv-1 Tar RNA". Tetrahedron 2004, 60, 8053-8064.
E. Riguet, J. Désiré, O. Boden, V. Ludwig, M. Göbel, C. Bailly, J.L. Décout. "Neamine dimers targeting the HIV-1 TAR RNA". Bioorg. Med. Chem. Lett. 2005, 15, 4651-4655.

## Revendications

1. Composition pharmaceutique comprenant un composé de formule : 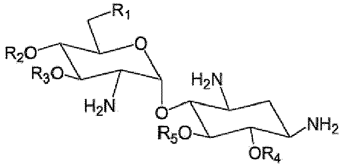 dans laquelle :
- R₁ = OH ou NH₂;
- R₂, R₃, R₄ et R_{5,} identiques ou différents, représentent : H ; un groupe alkyle, haloalkyle ou hétéroalkyle contenant entre 1 et 30 atomes de carbone en chaîne linéaire ou ramifiée ; ou un groupe alkaryle ou aralkyle contenant entre 1 et 30 atomes de carbone;
• si R₅ - H
- si R₂ = R₃ = R₄, alors R₂, R₃ et R₄ ≠H ;
- si R₂ = H, alors R₃ ≠ H et R₄ ≠ H ;
- si R₃ = H, alors R₂ ≠ H et R₂ ≠ H ;
- si R₄ = H, alors R₂ ≠ H et R₃ ≠ H ;
• si R₅ ≠ H
- si R₂ = H, alors R₃ ≠ H et R₄ ≠ H ;
- si R₃ = H, alors R₂ ≠ H et R₄ ≠ H ;
- si R₄ = H, alors R₂ ≠ H et R₃ ≠ H.

2. Composition selon la revendication 1 ***caractérisée* en ce que** R₂ et R₃, éventuellement R₄ et éventuellement R₅ sont identiques.

3. Composition selon l'une des revendications précédentes ***caractérisée* en ce que** R₂ et/ou R₃ et/ou R₄ et/ou R₅ sont choisis dans le groupe suivant : naphtyl-2-méthylène, naphtyl-1-méthylène, hexyle.

4. Composition selon l'une des revendications précédentes ***caractérisée* en ce que** sa formule est choisie dans le groupe suivant : 3',6-*O*,*O*'-di(naphtyl-2-méthylène) néamine ; 3',4',6-*O*,*O*',*O"*-tri(naphtyl-2-méthylène) néamine; 3',6-*O*,*O*'-di(naphtyl-1-méthylène) néamine; 3',4',*O*-*O*,*O*',*O"*-tri-*n*-hexyl néamine; 3',4'-*O*,*O*'-di-(naphtyl-2-méthyléne) néamine; 3',6-*O*,*O*'-di(naphtyl-2-propyl) néamine; 3',4',6-*O*,*O*',*O*"-tri(naphtyl-2-propyl) néamine ; 3',6-*O*,*O*'-di(naphtyl-2-butyl) néamine et 3',4',6-*O*,*O*',*O*"-tri(naphtyl-2-butyl) néamine.

5. Composition selon l'une des revendications précédentes, ***caractérisée* en ce qu'**elle comprend au moins un autre antibiotique tel qu'un β-lactate ou une fluoroquinolone.

6. Composition selon l'une des revendications précédentes pour son utilisation dans le traitement des infections bactériennes liées aux bactéries Gram positif telles que *Staphylococcus aureus* ou Gram négatif telles qu'*Escherichia coli,* notamment les souches résistantes aux aminoglycosides.

7. Procédé de préparation de composés tels que définis dans l'une des revendications 1 à 4 comprenant les étapes suivantes :
- protection des fonctions alcool et/ou amines en position 1, 3, 2' et 6', avantageusement par tritylation, sur les molécules paromamine ou néamine ;
- modification de la fonction hydroxyle en position 6 et/ou 3' ;
- déprotection, avantageusement par détritylation, des fonctions en positions 1, 3, 2' et 6'.

8. Procédé de préparation selon la revendication 7 ***caractérisé* en ce qu'**il s'agit d'un procédé biphasique se déroulant en présence d'un agent de transfert de phase.

9. Procédé de préparation selon la revendication 7 ***caractérisé* en ce que** la modification de la fonction hydroxyle en position 6 correspond à une réaction d'alkylation avec un composé RX, R étant défini comme R₂, R₃ et R₄, et X étant un halogène, avantageusement Cl, Br ou I, ou un groupement sulfonyle, avantageusement mésyl ou tosyl.

10. Procédé de préparation selon la revendication 9 ***caractérisé* en ce que** la réaction d'alkylation avec le composé RX a également lieu au niveau de la fonction hydroxyle en position 3', et éventuellement en position 4'.

11. Procédé de préparation selon la revendication 9 ***caractérisé* en ce que** la réaction d'alkylation avec le composé RX en position 6 est suivie par une réaction d'alkylation avec un composé R'X au niveau de la fonction hydroxyle en position 3', et éventuellement par une réaction d'alkylation avec un composé R"X au niveau de la fonction hydroxyle en position 4', R" et R' identiques ou différents étant identiques ou différents de R et étant définis comme R₂, R₃ et R₄.

12. Procédé de préparation selon la revendication 9 ***caractérisé* en ce que** la réaction d'alkylation avec le composé RX en position 6 est suivie par une protection de l'hydroxyle en position 3', avantageusement à l'aide d'un groupement Z, puis par une réaction d'alkylation avec le composé R'X au niveau de la fonction hydroxyle en position 4', R' étant identique ou différent de R et étant défini comme R₂, R₃ et R₄, le groupement Z étant acido-labile et introduit par réaction avec un dérivé halogéné, avantageusement Cl, Br ou I, ou sulfonylé, avantageusement mésyl ou tosyl, par exemple de type arylméthylène (Z = ArCH₂X, Ar = 2-naphtyl, 1-naphtyl, anthracényl, fluorényl, pyrényl...) ou silylé (Z = *tert*-butyldiméthylsilyl, triéthylsilyl...).

13. Procédé de préparation selon la revendication 8 ***caractérisé* en ce que** la modification de la fonction hydroxyle en position 6 correspond à une protection, avantageusement à l'aide d'un groupement Z, suivie d'une réaction d'alkylation avec le composé RX des fonctions hydroxyle en position 3' et en position 4', ou d'une réaction d'alkylation avec le composé RX de la fonction hydroxyle en position 3' suivie d'une seconde étape d'alkylation avec R'X de la fonction hydroxyle en position 4', R et R' étant définis comme R₂, R₃ et R₄, X étant un halogène, avantageusement Cl, Br ou I, ou un groupement sulfonyle, avantageusement mésyl ou tosyl, le groupement Z étant acido-labile et introduit par réaction avec un dérivé halogéné, avantageusement Cl, Br ou I, ou sulfonylé, avantageusement mésyl ou tosyl, par exemple de type arylméthylène (Z = ArCH₂X, Ar = -naphtyl, 1-naphtyl, anthracényl, fluorényl, pyrényl ...) ou silylé (Z = *tert*-butyldiméthylsilyl, triéthylsilyl...).

14. Procédé de préparation selon la revendication 7 ***caractérisé*** en ce l'étape de déprotection, notamment de détritylation en milieu acide, est mise en oeuvre pendant plusieurs heures, avantageusement 24 heures, de manière à éliminer le groupement R en position 6.

15. Procédé de préparation selon l'une des revendications 9 à 14 ***caractérisé* en ce que** une ou plusieurs réactions d'alkylation sont remplacées par des réactions d'arylation, d'acylation, de carbonatation, de carbamoylation, de sulfonylation ou d'aminosulfonylation.

## Patentansprüche

1. Pharmazeutische Zusammensetzung, umfassend eine Verbindung der Formel: 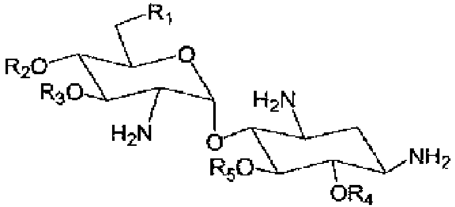 bei der
- R₁ = OH oder NH₂;
- R₂, R₃, R₄ und R₅, gleich oder verschieden, repräsentieren: H; eine Alkyl- , Haloalkyl- oder Heteroalkyl-Gruppe, umfassend zwischen 1 und 30 Kohlenstoffatome in linearer oder verzweigter Kette; oder eine Alkaryl- oder Aralkyl- Gruppe mit 1 bis 30 Kohlenstoffatome;
• wenn R₅ = H
- wenn R₂ = R₃ = R₄, dann R₂, R₃, und R₄ ≠ H;
- wenn R₂ = H, dann R₃ ≠ H und R₄ ≠ H;
- wenn R₃ = H, dann R₂ ≠ H und R₄ ≠ H;
- wenn R₄ = H, dann R₂ ≠ H und R₃ ≠ H;
• wenn R₅ ≠ H
- wenn R₂ = H, dann R₃ ≠ H und R₄ ≠ H;
- wenn R₃ = H, dann R₂ ≠ H und R₄ ≠ H;
- wenn R₄ = H, dann R₂ ≠ H und R₃ ≠ H.

2. Zusammensetzung gemäß Anspruch 1 ***dadurch gekennzeichnet, dass*** R₂ und R₃, eventuell R₄ und eventuell R₅ identisch sind.

3. Zusammensetzung gemäß einem der vorhergehenden Ansprüche ***dadurch gekennzeichnet, dass*** R₂ und/oder R₃ und/oder R₄ und/oder R₅ aus der folgenden Gruppe ausgewählt werden: Naphtyl-2-Methylen, Naphtyl-1-Methylen, Hexyl.

4. Zusammensetzung gemäß einem der vorhergehenden Ansprüche ***dadurch gekennzeichnet, dass*** die Formel aus der folgenden Gruppe ausgewählt wird: 3', 6-*O*, *O*'-di(naphtyl-2-methyl) Neamin ; 3', 4', 6- *O*, *O*', *O"*-tri(naphtyl-2-methylen) Neamin; 3', 6-*O*, *O*'- di(naphtyl-1-methyle Neamin ; 3', 4',6-*O*,*O*',*O*"-tri-n-hexyl-Neamin ; 3', 4'- *O*, *O*'- di-(naphtyl-2-methylen) Neamin ; 3', 6-*O*, *O*'-di(naphtyl-2-propyl) Neamin ; 3' , 4', 6-*O*, *O*', *O* "- tri(naphtyl-2-propyl) Neamin ; 3', 6- *O*, *O*'- di(naphtyl-2-butyl) Neamin und 3', 4', 6- *O*, *O*', *O*"-tri(naphtyl-2-butyl) Neamin.

5. Zusammensetzung nach einem der vorhergehenden Ansprüche, ***dadurch gekennzeichnet*, *dass*** sie mindestens ein weiteres Antibiotika enthält, wie ein β-Lactam oder ein Fluorochinolon.

6. Zusammensetzung nach einem der vorhergehenden Ansprüche, zur Verwendung bei der Behandlung bakterieller Infektionen durch Gram- positive Bakterien wie *Staphylococcus aureus,* oder Gram-negative wie *Escherichia coli,* insbesondere die Stämme, die gegen Aminoglykoside resistent sind.

7. Verfahren zur Herstellung von Verbindungen, wie definiert in einem der Ansprüche 1 bis 4, das die folgenden Schritte umfasst:
- Schutz der Alkohol- und/oder Aminfunktionen an der Position 1, 3, 2' und 6', möglichst durch Tritylierung, an den Paromamin- oder Neamin-Molekülen;
- Veränderung der Hydroxyl-Funktion an der Position 6 und/oder 3';
- Schutzgruppenentfernung, möglichst durch Detritylierung, der Funktionen an den Positionen 1, 3, 2' und 6'.

8. Herstellungsverfahren gemäß Anspruch 7, ***dadurch gekennzeichnet*, *dass*** es sich um ein zweiphasiges Verfahren handelt, das in Anwesenheit eines Phasentransferagents erfolgt.

9. Herstellungsverfahren gemäß Anspruch 7 ***dadurch gekennzeichnet*, *dass*** die Veränderung der Hydroxyl-Funktion an der Position 6 einer Alkylierungsreaktion mit einer RX - Verbindung entspricht, wobei R als R₂, R₃ und R₄ definiert ist und X ein Halogen, vorzugweise Cl, Br oder I, oder eine Sulfonyl-Gruppierung, vorzugweise Mesyl oder Tosyl, ist.

10. Herstellungsverfahren gemäß Anspruch 9 ***dadurch gekennzeichnet, dass*** die Alkylierungsreaktion mit der RX-Verbindung auch in Höhe der Hydroxyl-Funktion an der Position 3' und eventuell an Position 4' stattfindet.

11. Herstellungsverfahren gemäß Anspruch 9 ***dadurch gekennzeichnet*, *dass*** auf die Alkylierungsreaktion mit der RX-Verbindung an der Position 6 eine Alkylierungsreaktion mit einer R'X-Verbindung in Höhe der Hydroxyl-Funktion an der Position 3' und eventuell eine Alkylierungsreaktion mit einer R"X-Verbindung in Höhe der Hydroxyl-Funktion an der Position 4' folgt, wobei R" und R', gleich oder verschieden, gleich oder verschieden von R sind und als R₂, R₃ und R₄ definiert sind.

12. Herstellungsverfahren gemäß Anspruch 9, ***dadurch gekennzeichnet, dass*** auf die Alkylierungsreaktion mit der RX-Verbindung an der Position 6 ein Schutz des Hydroxyls an der Position 3', vorzugsweise mittels einer Z-Gruppierung, dann eine Alkylierungsreaktion mit der R'X-Verbindung in Höhe der Hydroxyl-Funktion an der Position 4' folgen, wobei R' dabei gleich oder verschieden von R ist und als R₂, R₃ und R₄ definiert ist, wobei die Z- Gruppierung säurelabil ist und durch Reaktion mit einem Halogen-Derivat, vorzugsweise Cl, Br oder I, oder einem Sulfonyl-Derivat, vorzugsweise Mesyl oder Tosyl, zum Beispiel vom Typ Arylmethylen (Z = ArCH₂X, Ar = 2-naphtyl, 1-naphtyl, Anthracenyl, Fluorenyl, Pyrenyl...) oder silyliert (Z = *tert*-butyldimethylsilyl, Triethylsilyl...) eingeführt wird.

13. Herstellungsverfahren gemäß Anspruch 8 ***dadurch gekennzeichnet*, *dass*** die Veränderung der Hydroxyl-Funktion an der Position 6 einem Schutz vorzugsweise mittels einer Z- Gruppierung entspricht, auf den eine Alkylierungsreaktion mit der RX-Verbindung der Hydroxyl-Funktionen an der Position 3' und der Position 4', oder eine Alkylierungsreaktion mit der RX-Verbindung der Hydroxyl-Funktion an der Position 3' folgt, die von einem zweiten Alkylierungsschritt mit R'X der Hydroxyl-Funktion an der Position 4' gefolgt wird, wobei R und R' dabei als R₂, R₃ und R₄ definiert sind, wobei X ein Halogen, vorzugweise Cl, Br oder I, oder eine Sulfonyl-Gruppierung, vorzugweise Mesyl oder Tosyl ist, wobei die Z-Gruppierung säurelabil ist und durch Reaktion mit einem Halogen-Derivat, vorzugsweise Cl, Br oder I oder Sulfonyl-Derivat, vorzugsweise Mesyl oder Tosyl, zum Beispiel vom Typ Arylmethylen (Z = ArCH₂X, Ar = 2- naphtyl, 1- naphtyl, Anthracenyl, Fluorenyl, Pyrenyl...) oder silyliert (Z = *tert*- butyldimethylsilyl ...) eingeführt wird.

14. Herstellungsverfahren gemäß Anspruch 7 ***dadurch gekennzeichnet*, *dass*** der Schritt der Schutzentfernung insbesondere der Detritylierung im sauren Milieu, über mehrere Stunden hinweg, vorzugsweise über 24 Stunden, durchgeführt wird, um die R-Gruppierung an der Position 6 zu entfernen.

15. Herstellungsverfahren gemäß einem der Ansprüche 9 bis 14 ***dadurch gekennzeichnet*, *dass*** eine oder mehrere Alkylierungsreaktionen durch Arylierungs-, Acylierungs-, Karbonisierungs-, Carbamoylierungs-, Sulfonylierungs- oder Amino-Sulfonylierungsreaktion ersetzt werden.

## Claims

1. A pharmaceutical composition comprising a compound with a formula: 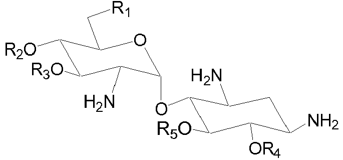 in which:
- R₁ = OH or NH₂;
- R₂, R₃, R₄ and R₅ being identical or different, are: H; an alkyl, haloalkyl or heteroalkyl group containing between 1 and 30 carbon atoms in a linear or branched chain; or an alkaryl or aralkyl group containing between 1 and 30 carbon atoms;
• if R₅ = H
- if R₂ = R₃ = R₄, then R₂, R₃ and R₄ ≠ H;
- if R₂ = H, then R₃ ≠ H and R₄ ≠ H;
- if R₃ = H, then R₂ ≠ H and R₄ ≠ H;
- if R₄ = H, then R₂ ≠ H and R₃ ≠ H ;
• if R₅
- if R₂ = H, then R₃ ≠ H and R₄ ≠ H;
- if R₃ = H, then R₂ ≠ H and R₄ ≠ H;
- if R₄ = H, then R₂ ≠ H and R₃ ≠ H.

2. Composition according to claim 1 ***characterised* in that** R₂ and R₃, possibly R₄ and possibly R₅, are identical.

3. Composition according to one of the previous claims ***characterised* in that** R₂ and/or R₃, and/or R₄ and/or R₅ are chosen from the following group: naphthyl-2-methylene, naphthyl-1-methylene and hexyl.

4. Composition according to one of the previous claims ***characterised* in that** the formula is selected from the following group: 3',6-*O*,*O*'-di(naphthyl-2-methylene) neamine; 3',4',6-*O*,*O*',*O*"-tri(naphthyl-2-methylene) neamine; 3',6-*O*,*O*'-di(naphthyl-1-methylene) neamine; 3',4',6-*O*,*O*',*O*"-tri-*n*-hexyl neamine; 3',4'-*O*,*O*'-di-(naphthyl-2-methylene) neamine; 3',6-*O*,*O*'-di(naphthyl-2-propyl) neamine; 3',4',6-*O*,*O*',*O*"-tri(naphthyl-2-propyl) neamine; 3',6-*O*,*O*'-di(naphthyl-2-butyl) neamine and 3',4',6-*O*,*O*',*O*"-tri(naphthyl-2-butyl) neamine.

5. - Composition according to one of the previous claims ***characterised* in that** it comprises at least one other antibiotic such as a β-lactam or a fluoroquinolone.

6. Composition according to one of the previous claims for use in the treatment of bacterial infections due to Gram positive bacteria such as *Staphylococcus aureus* or Gram negative bacteria such as *Escherichia coli,* particularly against aminoglycoside resistant strains.

7. Process for preparing compounds as defined in any of claims 1 to 4 comprising the following steps:
- protection, to advantage by tritylation, of alcohol and/or amine functional groups in the 1,3,2' and 6' positions on the paromamine or neamine molecules;
- modification of the hydroxyl functional group in the 6 and/or 3' positions;
- deprotection, to advantage by detritylation, of functional groups in the 1,3,2' and 6' positions.

8. Preparation process according to claim 7 ***characterised* in that** it is a biphasic process occurring in the presence of a phase transfer catalyst.

9. Preparation process according to claim 7 ***characterised* in that** the modification of the hydroxyl functional group in the 6 position is an alkylation reaction with a compound RX, R being defined as R₂, R₃ and R₄, and X being a halogen, to advantage Cl, Br or I, or a sulphonyl group, to advantage mesyl or tosyl.

10. Preparation process according to claim 9 ***characterised* in that** the alkylation reaction with the compound RX also occurs to the hydroxyl functional group in the 3' position, and possibly in the 4' position.

11. Preparation process according to claim 9 ***characterised* in that** the alkylation reaction with the compound RX in the 6 position is followed by an alkylation reaction with a compound R'X to the hydroxyl functional group in the 3' position, and possibly by an alkylation reaction with a compound R"X to the hydroxyl functional group in the 4' position, R" and R' being identical or different and being identical to or different from R and being defined as R₂, R₃ and R₄.

12. Preparation process according to claim 9 ***characterised* in that** the alkylation reaction with the compound RX in the 6 position is followed by protection of the hydroxyl in the 3' position, to advantage using a group Z, then by an alkylation reaction with the compound R'X to the hydroxyl functional group in the 4' position, R' being identical to or different from R and being defined as R₂, R₃ and R₄, the group Z being acid-labile and introduced by reaction with a halogenated derivative, to advantage Cl, Br or I, or a sulphonylated derivative, to advantage mesyl or tosyl, e.g. of the arylmethylene (Z = ArCH₂X, Ar = 2-naphthyl, 1-naphthyl, anthracenyl, fluorenyl, pyrenyl, etc.) or silylated (Z = *tert*-butyldimethylsilyl, triethylsilyl, etc.) type.

13. Preparation process according to claim 8 ***characterised* in that** the modification of the hydroxyl functional group in the 6 position corresponds to protection, to advantage using a group Z, followed by an alkylation reaction with the compound RX of the hydroxyl functional groups in the 3' and 4' positions, or an alkylation reaction with the compound RX of the hydroxyl functional group in the 3' position followed by a second alkylation step with R'X of the hydroxyl functional group in the 4' position, R and R' being defined as R₂, R₃ and R₄, X being a halogen, to advantage Cl, Br or I, or a sulphonyl group, to advantage mesyl or tosyl, the group Z being acid-labile and introduced by reaction with a halogenated derivative, to advantage Cl, Br or I, or a sulphonylated derivative, to advantage mesyl or tosyl, e.g. of the arylmethylene (Z = ArCH₂X, Ar = 2-naphthyl, 1-naphthyl, anthracenyl, fluorenyl, pyrenyl, etc.) or silylated (Z = *tert*-butyldimethylsilyl, triethylsilyl, etc.) type.

14. Preparation process according to claim 7 ***characterised* in that** the deprotection stage, particularly detritylation in an acid medium, occurs over several hours, to advantage 24 hours, in such a way as to eliminate the R group in the 6 position.

15. Preparation process according to one of claims 9 to 14 ***characterised* in that** one or more alkylation reactions are replaced by arylation, acylation, carbonatation, carbamoylation, sulfonylation or aminosulfonylation reactions.
